# EUROPEAN PATENT SPECIFICATION

(11) **EP 3 879 273 B1**
(45) Date of publication and mention of the grant of the patent: **12.06.2024**
(21) Application number: 20169813.1
(22) Date of filing: 16.04.2020
(51) Int. Cl.: G01N 33/84

(54) **POLYMER MATRIX FOR SENSING**
POLYMERMATRIX FÜR SENSOREN
MATRICE POLYMÈRE POUR LA DÉTECTION

(30) Priority: 10.03.2020 US 202016814643
(43) Date of publication of application: 15.09.2021
(73) Proprietor: SciLogica Corp., Denver CO 80206 (US)
(72) Inventor: BARWELL, Nicholas Paul, Coventry, Warwickshire CV5 8BN (GB); CRANE, Barry Colin, Oxon, OX15 6NF (GB)
(74) Representative: J A Kemp LLP

(56) References cited:
- US-A1- 2005 090 014
- US-A1- 2008 188 722
- XUDONG GE ET AL: "High-stability non-invasive autoclavable naked optical CO2 sensor", BIOSENSORS AND BIOELECTRONICS, vol. 18, no. 7, 1 July 2003 (2003-07-01), pages 857-865, XP055721667, AMSTERDAM, NL ISSN: 0956-5663, DOI: 10.1016/S0956-5663(02)00159-8
- ZHU ET AL: "A new ratiometric, planar fluorosensor for measuring high resolution, two-dimensional pCO"2 distributions in marine sediments", MARINE CHEMISTRY, ELSEVIER SCIENCE B.V., AMSTERDAM, NL, vol. 101, no. 1-2, 7 September 2006 (2006-09-07), pages 40-53, XP005457953, ISSN: 0304-4203
- Ekaterina V Skorb ET AL: "Layer-by-Layer Approach for Design of Chemical Sensors and Biosensors", Current Organic Chemistry, 1 June 2015 (2015-06-01), pages 1097-1116, XP055718870, Retrieved from the Internet: URL:https://www.ingentaconnect.com/content /ben/coc/2015/00000019/00000012/art00007?c rawler=true&casa_token=q-jXEjZz7HcAAAAA:oE vNKnRi5wU5DDCQn3QG8YgjOosQ2wfwVOM4q7UTM1Y1 Gr3JLLEGYxyuihIWZsd8WRVKtLY5XxBv-pI-So0HGO k [retrieved on 2020-07-29]

## Description

### FIELD OF THE INVENTION

The invention provides a polymer matrix for optical sensing of CO₂. A process for producing the polymer matrix is also described. The invention also provides an optical sensor for optical sensing of CO₂, comprising the polymer matrix of the invention. A method for making the optical sensor is also described. Also described herein is a method of measuring the CO₂ content of a sample, using the sensor of the invention.

### BACKGROUND TO THE INVENTION

It is important in many fields to be able to detect the presence, or indeed the precise concentration, of carbon dioxide in an aqueous sample. It is also useful to be able to accurately determine the pH of an aqueous sample. These quantities can frequently be assessed by the same methodology, because when CO₂ dissolves in an aqueous solution, some of it reacts with water to form an acidic species, carbonic acid (H₂CO₃). The presence of dissolved carbonic acid affects the pH of the solution. Thus, measurement of the pH of a solution not only yields the pH but also (subject to correction for the presence of any other acidic or basic species) the quantity of dissolved CO₂.

Detection of CO₂ in aqueous solution has most commonly been achieved with systems based on the Severinghaus electrode. The Severinghaus electrode comprises a buffer solution containing a reservoir of bicarbonate ions (HCO₃). The buffer solution is separated from a sample by a gas-permeable membrane. CO₂ diffuses into the buffer solution and equilibrates with the aqueous bicarbonate ions, thereby altering the pH of the buffer solution. A pH electrode monitors the pH of the buffer solution, and hence variations in the detected pH indicate variations in the quantity of CO₂ present in the sample.

This system is useful in CO₂ detection in procedures such as fermentation. However, the electrode-based system has major drawbacks for the monitoring of CO₂ in biological systems such as blood. In order to provide an accurate measurement, the Severinghaus electrode requires equilibration of the sample with the internal bicarbonate buffer. This means that the time taken to provide an accurate measurement is slow, typically around 5 to 15 minutes. However, in biological systems such as blood, CO₂ content and pH can vary suddenly, with catastrophic consequences to the patient. It is therefore desirable to provide a CO₂/pH detection system with a quicker response time. It is further desirable to provide a CO₂/pH detection system capable of continuously monitoring a sample, to provide real-time information.

Another disadvantage of the Severinghaus electrode in the field of blood gas sensing is that if the membrane separating the blood sample and the bicarbonate buffer bursts, bicarbonate can be released into the blood. The Severinghaus electrode is therefore unsuitable for measuring CO₂/pH of blood which is returned to the patient, as release of bicarbonate into the blood of a live patient can have damaging consequences.

Previous workers have attempted to provide sensors which overcome some of the above difficulties. Ge et al. have provided a so-called "naked optical CO₂ sensor" (Ge et al., Biosensors & Bioelectronics, 18 (2003), pp857-865). This device makes use of a fluorescent dye, pyranine, which has a pH-dependent fluorescence emission profile. The protonated form of pyranine has an emission peak which differs in wavelength from the deprotonated form of pyranine. Thus, the ratio of emission intensity at these two wavelengths can be used to determine the relative concentrations of the two forms of pyranine and hence the pH or CO₂ content of the system.

To avoid the need for a bicarbonate buffer solution, the fluorescent dye is suspended in a polymeric support together with a phase transfer agent. The phase transfer agent is typically a quaternary ammonium species that deprotonates the fluorescent dye and helps the fluorescent dye to dissolve. CO₂ diffuses into the polymeric support and, together with trapped water, generates carbonic acid, altering the pH inside the polymeric support and hence altering the ratio of deprotonated to protonated fluorescent dye.

However, there are considerable difficulties in manufacturing this optical sensor. The sensing region must be generated by producing a polymeric support and then suspending a fluorescent dye and a phase transfer agent within the matrix. The suspension is achieved by providing a solution comprising the fluorescent dye and the phase transfer agent. The polymeric support is then saturated with this solution and allowed to dry. However, the fluorescent dye, the polymer and the phase transfer agent typically have very different solubilities. It is therefore difficult to provide a solution containing the desired concentrations of each of the fluorescent dye and the phase transfer agent, and still more difficult to provide such a solution which can penetrate the polymeric support.

Not only is it difficult to combine these necessary components, but it is also difficult to provide a polymeric support/dye/phase transfer agent combination which is capable of retaining enough water to mediate pH variation in the presence of CO₂. One consequence of this is that the amount of water can be highly uncertain, leading to a lack of uniformity among sensors and considerable uncertainty in the concentrations of the dissolved dye and phase transfer agent. This renders accurate determination of CO₂ concentration more difficult. Another consequence is that the polymeric support, dye and phase transfer agent must be chosen carefully (Chu et al., Photonic Sensors, (2011) vol. 1, no. 3, pp234-250). The choice of polymeric support may be governed by solubility considerations rather than suitability for pH sensing (e.g. gas permeability, ion impermeability, and other relevant characteristics). Moreover, optimisation of the amount of fluorescent dye, phase transfer agent and water typically cannot be achieved.

Thus, there is evidently a need to provide a means for accurate pH or CO₂ sensing which has a rapid response time, can be used for continuous measurement and which overcomes the above manufacturing difficulties.

US 2005/0090014 describes a ratiometric fluorescent pH sensor for non-invasive, continuous monitoring of pH in such applications as fermentation processes. Also described in US 2005/0090014 are systems and methods to non-invasively and continuously monitor pH.

US 2008/0188722 describes an optical sensor capable of measuring two analytes simultaneously with a single indicator system.

The article "A new ratiometric, planar fluorosensor for meansuring high resolution, two-dimensional pCO2 distributions in marine sediments", Zhu et al., Marine Chemistry, vol 101, pp40-53, 2006, describes a sensor containing a mixture of the fluorophore HPTS, tetraoctylammonium cation and tetraoctylammonium hydroxide suspended within an ethyl cellulose membrane.

The article "Layer-by-layer approach for design of chemical sensors and biosensors", Skorb et al., Current Organic Chemistry, vol 19, pp1097-1116, 2015, is a review article considering the design and manufacturing of layered chemical sensors.

### SUMMARY OF THE INVENTION

The invention is defined in the claims.

The inventors have recognised that a counter-ion moiety, having a positive charge, may be used to form an ion pair with the fluorophore. The ion pair is subject to disruption by acid. Thus, the inventors have recognised that the "phase transfer agent" of the prior art can play a key role in optical sensing of CO₂, and they have consequently appreciated that the relative amounts of fluorophore and counter-ion moiety in the system are significant, and may be used to improve the accuracy of an optical sensor based on the ion pair and fluorophore.

Further, the inventors have noted that the difficulty of providing an optical sensor comprising the desired quantities of both the fluorophore and the counter-ion moiety can be reduced by covalently binding either the fluorophore or the counter-ion moiety to the polymer matrix. Where either the fluorophore or the counter-ion moiety is bound to the polymer matrix, only one of the fluorophore and the counter-ion moiety needs to be provided within the matrix by other means (e.g. suspended in the matrix). Thus, the need to perform the difficult task of providing a solution containing the desired concentrations of both the fluorophore and the oppositely-charged "phase transfer moiety" is entirely removed. One component may be suspended in the polymer matrix and this is considerably easier to achieve than suspending two components each having differing solubilities. Accordingly, the relative quantities of the fluorophore and the counter-ion moiety can be much more precisely controlled, leading to a more accurate sensor, as will be shown below.

Accordingly, herein is described a polymer matrix for optical sensing of CO₂, comprising a fluorophore, a positively charged counter-ion moiety and a polymeric support, wherein either the fluorophore or the counter-ion moiety is covalently bound to the polymeric support by a polymer linking moiety, and the molar ratio of counter-ion to fluorophore is 2:1 or greater.

Whichever of the fluorophore or the counter-ion moiety is not covalently bound to the polymeric support may be provided within the polymeric support by any means. Typically, whichever of the fluorophore or the counter-ion moiety is not covalently bound to the polymeric support by a polymer linking moiety is suspended in the polymeric support.

The polymer matrix of the invention can be made by any means. However, it is particularly convenient to provide a polymeric support having either the fluorophore or the counter-ion moiety covalently bound thereto, and then suspending the other component (i.e. whichever of the fluorophore and counter-ion moiety is not covalently bound to the polymeric support) in the polymeric support. The step of suspending either a fluorophore or a counter-ion moiety in the polymeric support is considerably easier, and leads to more accurately-known concentrations of the species suspended in the polymeric support, than prior art methods which require both the fluorophore and an oppositely-charged "phase transfer agent" to be suspended in a polymeric support. Accordingly, herein is described a method of producing a polymer matrix for optical sensing of CO₂, the method comprising:
(i) providing a polymeric support, wherein either a fluorophore or a counterion moiety is covalently bound to the polymeric support by a polymer linking moiety; and
(ii) suspending either a fluorophore or a counter-ion moiety, whichever is not covalently bound to the polymeric support via the polymer linking moiety, in the polymeric support.

The polymer matrix of the invention can be used to manufacture an optical sensor having improved accuracy and improved ease of manufacture. Thus, also described hereafter is an optical sensor for optical sensing of CO₂ comprising:
- a sensing region comprising a polymer matrix as described herein; and
- an optical waveguide arranged to direct light onto the sensing region.

In a particularly preferred embodiment of the invention, there is provided an optical sensor for optical sensing of CO₂, comprising:
- a sensing region comprising a polymer matrix as defined in the claims, wherein the polymeric support comprises a hydrophilic polymer; and the sensing region further comprises a gas-permeable hydrophobic membrane which is impermeable to liquids and hydrogen ions, and the sensor is configured to allow CO₂ to enter the sensing region through the hydrophobic membrane; and
- an optical waveguide arranged to direct light onto the sensing region.

This configuration of sensor is highly preferred as the hydrophobic membrane can prevent liquid water (and any acid or alkaline species therein) in an aqueous sample from entering the sensing region of the sensor. However, the hydrophobic membrane (which is typically gas-permeable and impermeable to liquids) permits gaseous CO₂ in the sample to diffuse into the sensing region. Thus, this configuration of sensor specifically detects CO₂.

The polymer matrix of the invention can be used to manufacture an optical sensor. Accordingly, described herein is a method of producing an optical sensor for optical sensing of CO₂, the method comprising:
(i) providing an optical waveguide;
(ii) disposing a polymer matrix as described herein on the optical waveguide.

In a particularly advantageous embodiment, the method of producing an optical sensor further comprises disposing a hydrophobic membrane on the polymer matrix. This can ensure that the sensor comprises a known and fixed quantity of water, and can be used for the specific detection of CO₂, as will be described hereafter.

The optical sensor of the invention can be used to detect and/or to quantify the amount of CO₂ in a sample. Accordingly, described herein is a method of measuring the CO₂ content of a sample, the method comprising:
(i) contacting an optical sensor as described herein with the sample;
(ii) providing excitation light to the sensing region through the optical waveguide; and
(iii) detecting the intensity I₁ of light emitted from the fluorophore at a first wavelength λ₁ through the optical waveguide.

### BRIEF DESCRIPTION OF THE FIGURES

Figure 1 is schematic diagram of the sensing chemistry on which the invention is based, illustrated using 8-hydroxy trisodium 1,3,6-pyrenetrisulfonic acid ("HTPS") as the fluorophore and cetyltrimethylammonium hydroxide ("CTMAOH⁺") as the positively-charged counter ion moiety.
Figure 2 contains two graphs indicating the emission spectrum of the fluorophore HTPS under various conditions. Graph A shows the emission spectrum of HTPS when CTMAOH⁺ is present at a molar ratio of 50:1 compared to HTPS, while Graph B shows the emission spectrum of HTPS when CTMAOH⁺ is present at a molar ratio of 25:1 compared to HTPS. In both cases, the spectrum in the presence of 0% CO₂ is shown (blue line; higher intensity at 470.0 nm) and 100% CO₂ (red line; lower intensity at 470.0 nm).
Figure 3 is a diagram of an optical sensor.
Figure 4 is a binding curve showing the binding of CO₂ to a polymer matrix in a sensor of the invention as a function of pCO₂.
Figure 5 is shows the actual pCO₂ values and pCO₂ values measured by a sensor according to the invention over a period of five hours.

### DETAILED DESCRIPTION OF THE INVENTION

Unless defined otherwise, all technical and scientific terms used herein have the same meanings as commonly understood by one of skill in the relevant art.

The invention is described hereafter with reference to particular embodiments and drawings. However, the invention is not limited to any specific embodiment or aspect of the following description, but is limited by the scope of the claims. Aspects or embodiments not falling within the scope of the claims are not part of the invention.

It should be noted that the following descriptions of various aspects of the invention are applicable to each of the differing embodiments of the invention. For instance, the description of the fluorophore concerns the fluorophore as present in the polymer matrix of the invention, and in the optical sensor of the invention, and in the various processes described herein.

### Definitions

As used in this specification and the appended claims, the singular forms "a", "an", and "the" include plural referents unless the content clearly dictates otherwise. Thus, for example, reference to "a counter-ion moiety" includes two or more counter-ion moieties.

Where the term "comprising" is used in the present description and claims, it does not exclude other elements or steps. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

"About" as used herein when referring to a measurable value such as an amount, a temporal duration, and the like, is meant to encompass variations of ± 20 % or ± 10 %, more preferably ± 5 %, even more preferably ± 1 %, and still more preferably ±0.1 % from the specified value, as such variations are appropriate to perform the disclosed methods.

"Alkyl" as used herein refers to monovalent straight-chained and branched alkyl groups. Typically, the alkyl group is a straight-chained alkyl group. An alkyl group may have from 1 to 30 carbon atoms (i.e. is a C₁₋₃₀ alkyl group). Typically, an alkyl group is a C₁₋₂₀ alkyl group or a C₁₋₁₀ alkyl group. Preferred alkyl groups include C₁₋₆ alkyl groups, for example C₁₋₄ alkyl groups. Examples of alkyl groups include methyl and ethyl groups, and straight-chained or branched propyl, butyl and pentyl groups. Particular alkyl groups include methyl, ethyl, n-propyl, isopropyl, n-butyl, sec-butyl, isobutyl, and tert-butyl groups.

"Cycloalkyl" as used herein refers to monovalent groups derived from a saturated monocyclic hydrocarbon. A cycloalkyl group may have, for instance, 3 to 12 carbon atoms but "cycloalkyl" typically refers to C₃₋₁₀ cycloalkyl groups. C₃₋₇ cycloalkyl groups are particularly preferred. Exemplary cycloalkyl groups include cyclopentyl and cyclohexyl groups.

"Alkenyl" as used herein refers to a monovalent hydrocarbon moiety comprising one or more carbon-carbon double bonds. Typically an alkenyl group contains one carbon-carbon double bond. The hydrocarbon moiety may be a straight-chain or branched; typically, the hydrocarbon moiety is a straight chain. An alkenyl group may have from 2 to 30 carbon atoms (i.e. is a C₂₋₃₀ alkenyl group). Typically, an alkenyl group is a C₂₋₂₀ or a C₂₋₁₀ alkenyl group. Preferred alkenyl groups include C₂₋₆ alkenyl groups, for example C₂₋₄ alkenyl groups.

"Cycloalkenyl" as used herein refers to non-aromatic monovalent groups derived from a monocyclic hydrocarbon comprising one or more carbon-carbon double bonds. Typically a cycloalkenyl group contains one carbon-carbon double bond. A cycloalkenyl group may have from 3 to 12 carbon atoms; "cycloalkenyl" typically refers to C₄₋₁₀ cycloalkenyl groups. C₅₋₇ cycloalkenyl groups are particularly preferred.

"Alkynyl" as used herein refers to a monovalent hydrocarbon moiety comprising one or more carbon-carbon triple bonds. Typically an alkynyl group contains one carbon-carbon triple bond. The hydrocarbon moiety may be a straight-chain or branched; typically, the hydrocarbon moiety is a straight chain. An alkynyl group may have from 2 to 30 carbon atoms (i.e. is a C₂₋₃₀ alkynyl group). Typically, an alkynyl group is a C₂₋₂₀ or a C₂₋₁₀ alkynyl group. Preferred alkynyl groups include C₂₋₆ alkynyl groups, for example C₂₋₄ alkynyl groups.

"Aryl" as used herein refers to a monovalent unsaturated aromatic carbocyclic group which may be monocyclic (for instance a phenyl group) or polycyclic, having multiple condensed rings (e.g. a naphthyl group). An aryl group typically contains from 6 to 14 carbon atoms. A preferred aryl group is phenyl.

"Alkylaryl" as used herein refers to a monovalent group comprising an alkylene and an aryl species as described herein. The number of carbon atoms in the alkyl moiety may be denoted by a prefix; for instance, a C₁₋₁₀ alkylaryl moiety comprises a C₁₋₁₀ alkylene group and an aryl group. The number of carbon atoms in the aryl moiety may also be denoted; for instance, a C₁₋₁₀ alkyl C₆₋₁₄ aryl species comprises a C₁₋₁₀ alkylene and a C₆₋₁₄ aryl species. A preferred alkylaryl moiety is benzyl.

"Heteroaryl" as used herein refers to monovalent aromatic carbocyclic groups having at least one heteroatom selected from oxygen, sulphur and nitrogen. Heteroaryl groups typically comprise one, two or three heteroatoms each independently selected from oxygen, sulphur and nitrogen, usually one such heteroatom. Heteroaryl groups may be monocyclic or polycyclic. A heteroaryl group typically comprises from 5 to 14 carbon atoms.

"Heterocycloalkyl" as used herein refers to monovalent saturated monocyclic rings comprising at least one heteroatom selected from oxygen, sulphur and nitrogen. Heterocycloalkyl groups typically comprise one, two or three heteroatoms each independently selected from oxygen, sulphur and nitrogen, usually one such heteroatom. Heterocycloalkyl rings may be monocyclic (e.g. piperidinyl) or polycyclic (e.g. decahydroquinoline). A heterocycloalkyl group typically comprises from 5 to 14 carbon atoms.

"Alkylene" as used herein refers to a divalent saturated hydrocarbon moiety which may be straight-chained or branched. Typically, the alkylene group is a straight-chained alkylene group. An alkylene group typically has from 1 to 10 carbon atoms (i.e. is a C₁₋₁₀ alkylene group). However, preferred alkylene groups include C₁₋₆ alkylene groups, for example C₁₋₄ alkylene groups. Examples of alkyl groups include methylene (-CH₂-) and ethylene (-CH₂CH₂-) groups.

"Alkenylene" as used herein refers to a divalent hydrocarbon moiety comprising one or more carbon-carbon double bonds. Typically an alkenylene group contains one carbon-carbon double bond. The hydrocarbon moiety may be a straight-chain or branched; typically, the hydrocarbon moiety is a straight chain. An alkenylene group typically has from 2 to 10 carbon atoms (i.e. is a C₂₋₁₀ alkenylene group). However, preferred alkenylene groups include C₂₋₆ alkenylene groups, for example C₂₋₄ alkenylene groups.

"Alkynylene" as used herein refers to a divalent hydrocarbon moiety comprising one or more carbon-carbon triple bonds. Typically an alkynylene group contains one carbon-carbon triple bond. The hydrocarbon moiety may be a straight-chain or branched; typically, the hydrocarbon moiety is a straight chain. An alkynylene group typically has from 2 to 10 carbon atoms (i.e. is a C₂₋₁₀ alkenylene group). However, preferred alkynylene groups include C₂₋₆ alkynylene groups, for example C₂₋₄ alkynylene groups.

The term "halogen" as used herein is intended to include fluorine, chlorine, bromine and iodine atoms, typically fluorine, chlorine or bromine.

The term "oxo" indicates a =O group. The term "hydroxy" indicates an -OH group.

Reference herein to a group in its protonated form should be taken to encompass reference to any deprotonated form which may be produced in solution. For instance, reference to "hydroxy" or "-COOH" should be taken to encompass such groups when deprotonated to -O⁻ or -COO⁻ in solution.

### Sensing chemistry

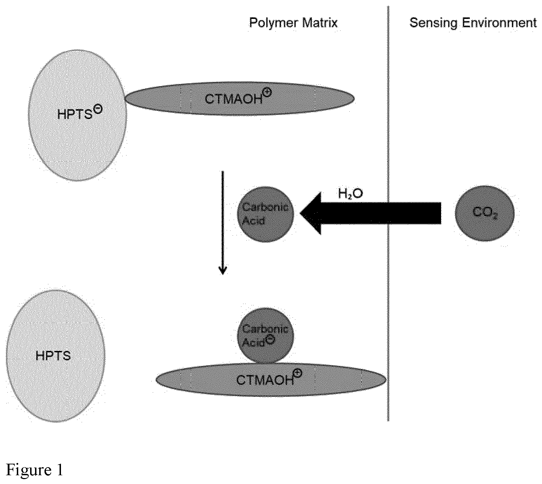

The sensing chemistry employed in the invention is illustrated in Figure 1. The polymer matrix of the invention comprises a fluorophore which, when deprotonated, carries a negative charge; and a positively charged counter-ion moiety. For the purposes of illustration, deprotonated HTPS (hydroxypyrene trisulfonate) and the quaternary ammonium cation CTMAOH⁺ (CetylTriMethylAmmonium) are shown in Figure 1. These two oppositely charged ionic species interact, forming an ion pair.

When CO₂ enters the sensing environment, it interacts with water trapped in the polymer matrix. The reaction of H₂O and CO₂ generates carbonic acid, H₂CO₃. Carbonic acid interacts with the ion pair, by protonating the fluorophore. This leaves a hydrogencarbonate anion, HCO₃-, which interacts with the positively-charged counterion moiety, forming a new ion pair. By way of example, in Figure 1, interaction with carbonic acid causes deprotonated HTPS to become protonated, leaving a new ion pair comprising HCO₃⁻ and CTMAOH⁺.

This interaction causes a change in the emission spectrum and/or the absorption spectrum of the system. The deprotonated fluorophore has a differing fluorescence emission spectrum to that of the protonated fluorophore. Similarly, the deprotonated fluorophore has a differing absorption spectrum to that of the protonated fluorophore. By way of example, deprotonated HTPS absorbs and emits most strongly at wavelengths of around 465 nm, while protonated HTPS absorbs and emits most strongly around 405 nm.

The polymeric matrix of the invention is therefore useful in a sensing method for optical sensing of CO₂, wherein the sensing method involves disruption of an ion pair comprising the fluorophore and the counter-ion moiety by protonation of the negatively-charged moiety of the fluorophore.

Thus, monitoring the absorption spectrum or the emission spectrum of the polymer matrix will show an increase in absorption strength or emission intensity of the spectral peak corresponding to the protonated form of the fluorophore as the CO₂ concentration within the polymer matrix increases. The absorption strength or emission intensity of the spectral peak corresponding to the deprotonated form of the fluorophore will correspondingly decrease. Similarly, an increase will be observed in absorption strength or emission intensity of the spectral peak corresponding to the deprotonated form of the fluorophore as the CO₂ concentration within the polymer matrix decreases.

For reference purposes, it is noted that similar chemistry occurs in the measurement of pH. In the presence of an H⁺ or H₃O⁺ ion provided by an acid, the negatively-charged fluorophore may become protonated. The concentration of H⁺ or H₃O⁺ ions varies with pH, increasing as pH decreases. Thus, as pH decreases, the absorption spectrum or the emission spectrum of a fluorophore in a polymer matrix will show an increase in absorption or emission intensity of the spectral peak corresponding to the protonated form of the fluorophore. Similarly, a decrease will be seen in the absorption or emission intensity of the spectral peak corresponding to the deprotonated form of the fluorophore.

However, sensing of pH relies on the deprotonation/protonation of the fluorophore. Accordingly, pH sensing methods and uses do not require the presence of a positively-charged counter-ion moiety. Thus, the polymeric matrix is particularly suited to CO₂ sensing. The invention provides optical sensors for detection of CO₂, and also described herein are methods of detecting or quantifying the CO₂ content of a sample.

It should be noted that in some cases, either the protonated or the deprotonated form of the fluorophore may have a very weak absorption or emission spectrum. In such cases, it is possible that no increase or decrease in the absorption strength or emission intensity of the spectral peak corresponding to that form of the fluorophore may be observed as its concentration varies. Rather, only the increase or decrease in absorption strength or emission intensity of the spectral peak corresponding to the other form of the fluorophore may be observed.

The positively-charged counter-ion moiety is generally used in excess. The more positively-charged counter-ion moiety is present, the more likely it is that each fluorophore moiety will form an ion pair with the counter-ion moiety in the absence of CO₂. This increases the spectral change that occurs when CO₂ enters the polymer matrix, as the fluorophores which become protonated (and counter-ion moieties which become associated with a carbonate ion) are more likely to have been previously part of an ion pair comprising a fluorophore and a counter-ion. If the fluorophore does not form an ion pair with the counter-ion moiety in the absence of CO₂, then it is likely to remain protonated and the effect of protonation will not be observed when CO₂ is added to the system.

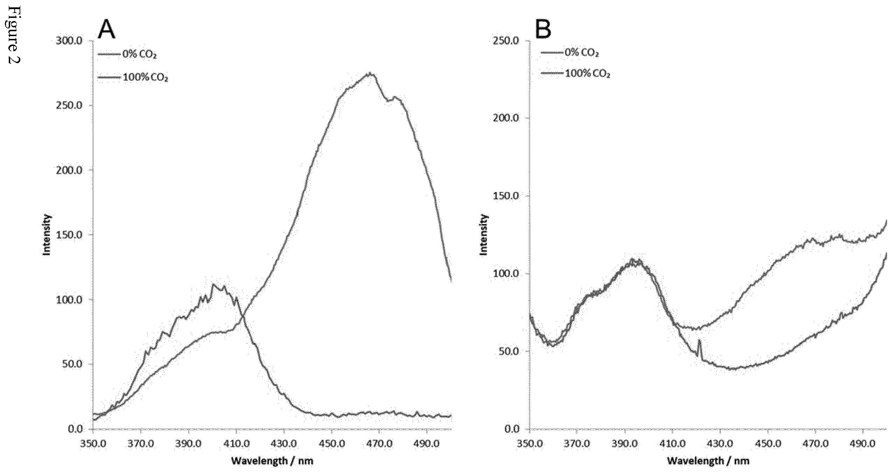

This is illustrated in Figure 2. Graph A of Figure 2 shows the emission spectrum of HTPS when CTMAOH⁺ is present at a molar ratio of 50:1 compared to HTPS, while Graph B shows the emission spectrum of HTPS when CTMAOH⁺ is present at a molar ratio of 25:1 compared to HTPS. In both cases, the spectrum in the presence of 0% CO₂ is shown (blue line; higher intensity at 470.0 nm) and 100% CO₂ (red line; lower intensity at 470.0 nm). It can be clearly seen that, where the counter-ion (CTMAOH⁺):fluorophore (HTPS) ratio is larger, i.e. 50:1 rather than 25:1, a greater intensity change is observed upon the addition of CO₂. The greater intensity change means that the sensor is more sensitive and hence has better performance.

The polymer matrix of the invention is therefore particularly advantageous as large quantities of the counter-ion moiety may easily be incorporated into the polymeric support, ensuring that the counter-ion moiety is in excess. Thus, in a preferred embodiment, the counter-ion moiety is covalently bound to the polymeric support. Moreover, the counter-ion moiety is present in a molar excess compared to the fluorophore, the molar ratio of counter-ion to fluorophore being 2:1 or greater. In a particularly preferred embodiment, the counter-ion moiety is covalently bound to the polymeric support (as well as being present in a molar excess compared to the fluorophore).

For example, the polymeric matrix may comprise up to about 25% by weight of the counter-ion moiety, typically up to about 20% by weight, preferably up to about 10% by weight of the counter-ion moiety. The polymeric matrix may therefore comprise about 1-25% by weight of the counter-ion moiety, typically about 2-20% by weight and preferably about 5-10% by weight of the counter-ion moiety. Where the counter-ion moiety is incorporated into the polymeric support, the polymeric support may comprise up to about 25% by weight of the counter-ion moiety, typically up to about 20% by weight and preferably up to about 10% by weight of the counter-ion moiety. The polymeric support may therefore comprise about 1-25% by weight of the counter-ion moiety, typically about 2-20% by weight and preferably about 5-10% by weight of the counter-ion moiety.

By contrast, the polymer matrix of the invention may comprise up to about 10% by weight of the fluorophore, typically up to about 7% by weight, preferably up to about 5% by weight of the fluorophore. The polymeric matrix may therefore comprise about 0.1-10% by weight of the fluorophore, preferably about 1-5% by weight of the fluorophore, e.g. about 2%, 3% or 4% by weight of the fluorophore. Where the fluorophore is incorporated into the polymeric support, the polymeric support may comprise up to about 10% by weight of the fluorophore, typically up to about 5% by weight of the fluorophore. For example, the polymeric support may comprise about 0.1-10% by weight of the fluorophore, preferably about 1-5% by weight of the fluorophore, e.g. about 2%, 3% or 4% by weight of the fluorophore.

The molar ratio of counter-ion to fluorophore is 2:1 or greater. Typically, the molar ratio of counter-ion to fluorophore is at least 20:1. Preferably, the molar ratio of counter-ion to fluorophore is at least 30:1, for example at least 40:1 or at least 50:1. Generally, the molar ratio of counter-ion to fluorophore is from 20:1 to 1000:1, preferably from 30:1 to 500:1 or 40:1 to 200:1. By way of example, the molar ratio of counter-ion to fluorophore may be about 50:1 or about 100: 1.

### Fluorophore

### I. Fluorescence emission and absorption characteristics

A fluorophore is a moiety which can absorb light and emit light by fluorescent emission.

Usually, the fluorophore described herein absorbs light in the visible region of the electromagnetic spectrum. Said fluorophore also usually emits light in the visible region of the electromagnetic spectrum. By "the visible region of the electromagnetic spectrum" is meant electromagnetic radiation having a wavelength of from about 400 nm to about 700 nm. The fluorophore may also absorb and/or emit radiation outside the visible region of the electromagnetic spectrum.

The fluorophore can exist in deprotonated and protonated forms. Protonation of a fluorophore affects its electronic structure. Consequently, the protonated and deprotonated forms of a fluorophore will typically absorb and emit at different wavelengths. The characteristic emission and absorption spectra of the protonated form and the deprotonated form differ. Thus, measuring the absorption or emission spectrum of a fluorophore will indicate whether the protonated form, the deprotonated form or a mixture of protonated and deprotonated forms are present.

Of course, where a fluorophore has multiple different protonated and deprotonated forms (for instance, where a fluorophore has two or more labile protons), measuring the absorption or emission spectrum of the fluorophore can indicate which of the multiple different protonated and deprotonated forms are present. Thus, reference to "the protonated form of the fluorophore" and "the deprotonated form of the fluorophore" should be understood as referring to "one of the protonated forms of the fluorophore" and "one of the deprotonated forms of the fluorophore" in cases where a fluorophore has more than one protonated form and more than one deprotonated form.

Usually, therefore, the fluorophore has at least one protonated form and at least one deprotonated form, wherein the fluorescence emission spectrum of the protonated form differs from the fluorescence emission spectrum of the deprotonated form. For example, the fluorescence emission spectrum of the protonated form may have a peak emission wavelength of λ₁ and the fluorescence emission spectrum of the deprotonated form may have a peak emission wavelength of λ₂, wherein λ₁ differs from λ₂. Typically, λ₁ differs from λ₂ by at least 5 nm; and preferably λ₁ differs from λ₂ by at least 10 nm or at least 20 nm. The larger the difference between λ₁ and λ₂, the easier it is to distinguish the two peaks in a fluorescence emission spectrum.

Similarly, in another preferred embodiment, the absorption spectrum of the protonated form differs from the absorption spectrum of the deprotonated form of the fluorophore. For example, the absorption spectrum of the protonated form may have a peak absorption wavelength of λ₃ and the absorption spectrum of the deprotonated form may have a peak absorption wavelength of λ₄, wherein λ₃ differs from λ₄. Typically, λ₃ differs from λ₄ by at least 5 nm; and preferably λ₃ differs from λ₄ by at least 10 nm or at least 20 nm. The larger the difference between λ₃ and λ₄, the easier it is to distinguish the two peaks in an absorption spectrum.

Thus, the fluorophore has a pH-dependent absorption spectrum and/or a pH-dependent fluorescent emission spectrum. By "a pH-dependent absorption spectrum" is meant that the absorption spectrum comprises (a) an absorption peak corresponding to the absorption of a protonated form of the fluorophore; and/or (b) an absorption peak corresponding to the absorption of a deprotonated form of the fluorophore. By "a pH-dependent fluorescent emission spectrum" is meant that the fluorescence emission spectrum comprises (a) an emission peak corresponding to the emission of a protonated form of the fluorophore; and/or (b) an emission peak corresponding to the emission of a deprotonated form of the fluorophore. Preferably, the fluorophore has a pH-dependent absorption spectrum or a pH-dependent fluorescent emission spectrum comprising both (a) and (b), as this permits ratiometric measurements of the two forms.

As the concentration of carbonic acid in the polymer matrix increases, the proportion of fluorophore which adopts the protonated form will increase. Consequently, the intensity of absorption or emission spectral peaks associated with the protonated form of the fluorophore will increase.

Typically, therefore, the emission spectrum of the fluorophore comprises (a) an emission peak corresponding the fluorescence emission of a protonated form of the fluorophore, the intensity of which is correlated to the concentration of the protonated fluorophore in the polymer matrix. Similarly, the emission spectrum of the fluorophore typically also or alternatively comprises (b) an emission peak corresponding to the fluorescence emission of a deprotonated form of the fluorophore, the intensity of which is correlated to the concentration of the deprotonated form of the fluorophore in the polymer matrix. Preferably, the emission spectrum comprises (a) and (b).

Similarly, the absorption spectrum of the fluorophore typically comprises (a) an absorption peak corresponding the absorption of a protonated form of the fluorophore, the intensity of which is correlated to the concentration of the protonated fluorophore in the polymer matrix. Similarly, the absorption spectrum of the fluorophore typically also or alternatively comprises (b) an absorption peak corresponding to the absorption of a deprotonated form of the fluorophore, the intensity of which is correlated to the concentration of the deprotonated form of the fluorophore in the polymer matrix. Preferably, the absorption spectrum comprises (a) and (b).

Where the intensity of an absorption or emission peak is correlated to the concentration of a protonated or deprotonated form of the fluorophore, the concentration of that species (and hence the CO₂ content of the system) may be calculated using the intensity of that peak alone. However, a more accurate measurement may be obtained using a ratiometric approach. This is possible where the absorption or emission spectrum comprises two peaks, one corresponding to the protonated form and another corresponding to the deprotonated form of the fluorophore. Where the ratio of intensity of these two peaks is obtained and used to calculate the CO₂ content of the system, errors in the calculation due to photobleaching of the fluorophore can be eliminated.

Thus, in a preferred embodiment, the fluorophore can undergo fluorescence emission at a first wavelength λ₁ with an intensity I₁, and can undergo fluorescence emission at a second wavelength λ₂ with an intensity I₂, wherein the ratio of I₁ to I₂ varies dependent upon pH. In a similarly preferred embodiment, the fluorophore can absorb light at a first wavelength λ₃ with an intensity I₃, and can absorb light at a second wavelength λ₄ with an intensity I₄, wherein the ratio of I₃ to I₄ varies dependent upon pH.

Generally, fluorescence emission at λ₁ is the fluorescence emission of the protonated form of the fluorophore, while fluorescence emission at λ₂ is the fluorescence emission of the deprotonated form of the fluorophore. Similarly, absorption at λ₃ is the absorption of the protonated form of the fluorophore, while absorption at λ₄ is the absorption of the deprotonated form of the fluorophore. These numeric values are merely labels and may of course be reversed.

### II. Protonated, deprotonated and salt forms of fluorophore

The fluorophore has a protonated and deprotonated form and so may interact with water or water vapour to gain or lose one or more protons. A system comprising H₂O and the fluorophore will tend towards an equilibrium wherein the deprotonated and/or the protonated forms of the fluorophore are present.

How much of each of these forms are present (i.e. the position of the equilibrium) will depend on the pH of the system and the pKa of the fluorophore moiety. Where the system is highly acidic, generally all of the fluorophore will be protonated; where the system is highly alkaline, generally all of the fluorophore will be deprotonated. In biological systems, the pH is generally neither strongly acidic nor strongly alkaline. A fluorophore may be selected having a suitable pKa for its intended use.

Sensing will be most efficacious where the pKa of the fluorophore is similar to the pH of the environment in which it is to be used. Where the pKa of the fluorophore is highly dissimilar from the pH of the environment in which it is used, almost all of the fluorophore will remain protonated or deprotonated when the pH of the environment changes. Thus, the variation in the absorption and/or emission properties of the system will be small and the difference will be difficult to detect using optical methodology. However, where the pKa of the fluorophore is similar to the pH of its environment, small changes in the pH of the environment can cause a significant change in the relative concentrations of the protonated and deprotonated forms of the fluorophore. Thus, the absorption and/or emission properties of the system will change more significantly and are easier to detect via the absorption or emission spectrum of the system.

The polymer matrix may be useful in optical detection in biological systems. Biological systems generally have a near-neutral pH. Near-neutral pH is typically pH 5.5 to 8.5, in particular pH 6 to pH 8. Typically, therefore, the fluorophore has a pKa of from about 3.5 to 10. In a preferred embodiment, the fluorophore has a pKa of from about 4 to 9, for example from 5 to 8 or 6 to 8.

In some embodiments, the fluorophore comprises a weak acid or a salt of a weak acid. In the context of the present invention, a "weak acid" refers to an acid having a pKa of from 5 to 9, for instance from 5.5 to 8.5, preferably from 6 to 8 or from 6.5 to 8.

Thus, the fluorophore described herein may be any fluorophore which carries a negatively-charged moiety at near-neutral pH. The negatively charged moiety can be stabilised by the counter-ion moiety. However, the fluorophore can also become protonated (e.g. in the presence of acid). The fluorophore carrying a negatively charged moiety will typically exist in an equilibrium which also comprises the protonated form of the fluorophore at near-neutral pH. Preferably, the position of equilibrium lies towards the deprotonated form of the fluorophore at near-neutral pH. For instance, 50% or more of the fluorophore may be deprotonated (i.e. comprise a negatively-charged moiety) at near-neutral pH.

Preferably, the fluorophore comprises an -O⁻ group and/or a -COO⁻ group at near-neutral pH. Of course, the fluorophore comprising an -O⁻ group or -COO⁻ group may exist in equilibrium with a form of the fluorophore wherein the -O⁻ group and/or the -COO⁻ is protonated.

Fluorophores having multiple deprotonated forms are possible, such as fluorophores having multiple charged moieties able to accept a proton. Reference to a fluorophore carrying a charge or a charged moiety does not exclude the possibility that the fluorophore may include further charged moieties capable of accepting a proton. Further, reference to a "fluorophore" herein should be taken to encompass both the deprotonated form of the fluorophore and the protonated form of the fluorophore unless indicated otherwise.

Where the fluorophore is in salt form, for instance where the fluorophore comprises a salt of a weak acid, the fluorophore may include one or more positive ions. These positively-charged ions are not covalently bound to the fluorophore or the polymer matrix. Such ions are dissociated from the fluorophore when the fluorophore is in solution. The nature of the positive ion is not particularly limited. Suitable positive ions include cations of elements in Group I or Group II of the periodic table (e.g. Li⁺, Na⁺, K⁺, Mg²⁺ or Ca²⁺) or ammonium salts (e.g. NH₄⁺, CH₃NH₃⁺ or CH₃CH₂NH₃⁺). Na⁺ and K⁺ are preferred.

As the fluorophore may comprise a positive ion or ions, the overall charge of the fluorophore is not necessarily negative, even in its deprotonated form. However, positive ions need not be present; for instance, if the polymeric support is generated having a fluorophore covalently bound thereto and then washed, positive ions associated with the fluorophore may be removed. The overall charge of the fluorophore may therefore be positive, neutral or negative. Typically, the overall charge of the fluorophore is neutral or negative.

### III. Structure of fluorophore

The fluorophore has at least a protonated form and a deprotonated form. At near-neutral pH, the fluorophore comprises a negatively-charged moiety which can accept a proton. Beyond this, the chemical structure of the fluorophore moiety is not particularly limited. The fluorophore may or may not be covalently bound to the polymeric support. For instance, in one embodiment the fluorophore may consist of the fluorophore moiety and optionally one or more associated ions; in another embodiment the fluorophore may be covalently attached to the polymeric support. It is preferred that the fluorophore is not attached covalently bound to the polymeric support via a polymer linking moiety.

It is preferred that the fluorophore can be dissolved in a coating solution; a coating solution in which the fluorophore is dissolved can be added to the polymeric matrix in order to suspend the fluorophore in the polymer matrix. A coating solution may comprise any solvent, for instance water and/or an organic solvent.

An important feature of the fluorophore moiety is that it is typically stable in aqueous solution. It is also preferred that the fluorophore is resistant to photobleaching, as this allows the polymer matrix to be used repeatedly and/or for long periods of time. It is particularly preferred that the fluorophore is resistant to photobleaching over long periods, as this allows the polymer matrix to be used in sensing applications for continuous measurements over long periods. Typically, therefore, loss of fluorophore due to photobleaching is less than 5% per hour of irradiation with visible light, preferably less than 2% or less than 1%. It is particularly preferred that loss of fluorophore due to photobleaching is less than 5% per 24 hours of irradiation with visible light, preferably less than 2% or less than 1%.

Preferably, the deprotonated form of the fluorophore comprises an -O⁻ moiety or a - COO⁻ moiety. These species are preferred as the highly electronegative O atom can stabilise the negative charge of the deprotonated fluorophore. When protonated, therefore, it is preferred that the fluorophore comprises an -OH moiety or a -COOH moiety. It is particularly preferred that the deprotonated fluorophore comprises an -O⁻ moiety, which can be protonated to form an -OH moiety.

Further, the fluorophore typically comprises an extended system of delocalised electrons, as such systems typically give rise to the excited electronic states needed to allow absorption and fluorescence in the visible region of the electromagnetic spectrum.

In a preferred embodiment, therefore, the fluorophore comprises an optionally substituted polycyclic aryl or polycyclic heteroaryl species, substituted with at least one -OH or -COOH group. Reference to an -OH or -COOH group refers to the fluorophore in its protonated form; the deprotonated form of the fluorophore would comprise an -O⁻ moiety or a -COO⁻ moiety. In a particularly preferred embodiment, the fluorophore comprises an optionally substituted polycyclic aryl or polycyclic heteroaryl species, substituted with at least one -OH group (again, reference to an -OH group concerns the fluorophore in its protonated form).

In some embodiments, the fluorophore is pyranine or a derivative thereof. Pyranine (also referred to as HTPS or 8-hydroxy trisodium 1,3,6-pyrenetrisulfonic acid) has the following structure:

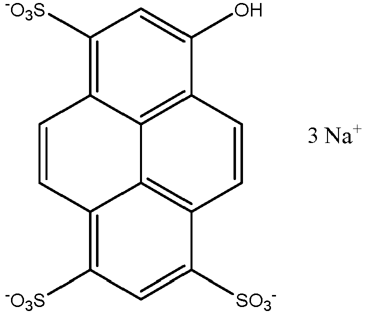

The -OH moiety can be deprotonated, yielding a deprotonated fluorophore having an absorption and emission spectrum which differ from the absorption and emission spectra of the protonated form. In some embodiments, therefore, the fluorophore comprises or is pyranine.

Modified versions of pyranine are also known to be useful as fluorophores in pH sensing. One example of such a compound is modified DHDS (dihydroxy pyrene disulphonic acid), shown below.

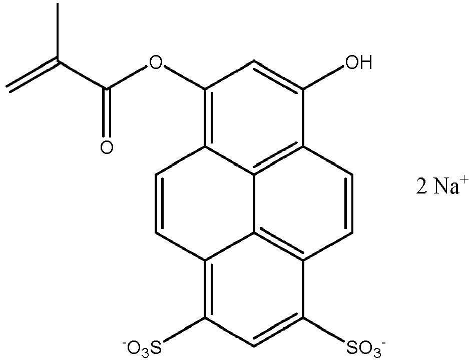

Thus, in some embodiments the fluorophore comprises a species of formula (Fl-A):

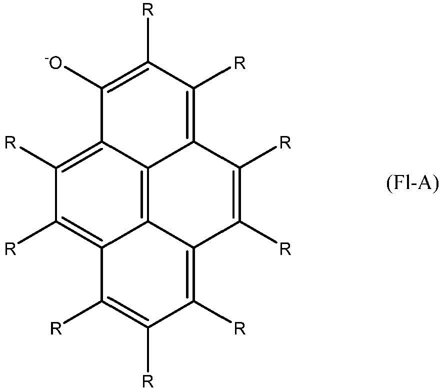

Each R group is independently selected from H, halogen, cyano, nitro, hydroxy, C₁₋₁₀ alkyl, C₃₋₁₀ cycloalkyl, C₄₋₁₀ cycloalkenyl, aryl, alkylaryl, heteroaryl, heterocycloalkyl, C₂₋₁₀ alkenyl, C₂₋₁₀ alkynyl, -SR^{a}, -OR^{a}, -SO₂R^{a}, -SO₃⁻, -SOR^{a}, -SO₂NR^{a}, -S(O)(NR^{a})R^{a}, -NR^{a}₂, - NR^{a}COR^{a}, -NR^{a}CO₂R^{a}, -COR^{a}, -CO₂R^{a}, -CONR^{a}₂, or a covalent bond attaching the fluorophore to the polymer linking moiety; or two neighbouring R groups are joined together to form a carbocyclic or heterocyclic ring.

In some embodiments, each R group is independently selected from H, halogen, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -SR^{a}, -OR^{a}, -SO₂R^{a}, -SO₃⁻, -SOR^{a}, -SO₂NR^{a}, -S(O)(NR^{a})R^{a}, -NR^{a}₂, -NR^{a}COR^{a}, - NR^{a}CO₂R^{a}, -COR^{a}, -CO₂R^{a}, and -CONR^{a}₂. For example, each R may be preferably be independently selected from H, hydroxy, C₁₋₆ alkyl, C₂₋₆ alkenyl, -OR^{a}, -SO₃⁻, -COR^{a}, and - CO₂R^{a}. Particularly preferably, each R is independently selected from H, C₁₋₄ alkyl, and - SO₃⁻.

Each R^{a} group is independently selected from H, C₁₋₁₀ alkyl, C₃₋₁₀ cycloalkyl, C₄₋₁₀ cycloalkenyl, aryl, alkylaryl, heteroaryl, heterocycloalkyl, C₂₋₁₀ alkenyl, and C₂₋₁₀ alkynyl. Preferably, each R^{a} is independently selected from H, C₁₋₁₀ alkyl, C₃₋₆ cycloalkyl, C₄₋₆ cycloalkenyl, and C₂₋₁₀ alkenyl. Particularly preferably, each R^{a} is independently selected from Hand C₁₋₄ alkyl.

Any R group capable of substitution may be optionally substituted by one or more substituents. The one or more substituents may be present at any suitable position on the R group, including (where present) on an R^{a} group. The one or more substituents are typically selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -OR^{b}, -NR^{b}₂, -COR^{b}, -CO₂R^{b}, and -CONR^{b}₂. Each R is usually substituted by 0, 1, 2 or 3 substituents, preferably by 0 or 1 substituents. Preferred substituents include halogen, oxo, hydroxy, C₁₋₆ alkyl, C₃₋₆ cycloalkyl, C₂₋₆ alkenyl, -OR^{b}, -NR^{b}₂, -COR^{b}, and -CO₂R^{b}. Particularly preferred substituents include halogen, hydroxy, C₁₋₄ alkyl, -OR^{b}, -NR^{b}₂, -COR^{b}, and -CO₂R^{b}.

R^{b} is selected from H, C₁₋₆ alkyl, C₃₋₆ cycloalkyl, C₄₋₆ cycloalkenyl, and C₂₋₆ alkenyl. Preferably, R^{b} is selected from H, C₁₋₆ alkyl, C₃₋₆ cycloalkyl, and C₂₋₆ alkenyl. More preferably, R^{b} is selected from Hand C₁₋₄ alkyl.

In a preferred embodiment, therefore, each R group is independently selected from H, hydroxy, C₁₋₆ alkyl, C₂₋₆ alkenyl, -OR^{a}, -SO₃⁻, -COR^{a}, and -CO₂R^{a} and each R^{a} is independently selected from H, C₁₋₁₀ alkyl, C₃₋₆ cycloalkyl, C₄₋₆ cycloalkenyl, and C₂₋₁₀ alkenyl. In this embodiment, each R may be unsubstituted or substituted by one substituent independently selected from halogen, hydroxy, C₁₋₄ alkyl, -OR^{b}, -NR^{b}₂, -COR^{b}, and -CO₂R^{b} wherein R^{b} is selected from Hand C₁₋₄ alkyl.

Where an R group, or a substituent thereon, comprises a hydroxy group or a -COOH group, reference to "hydroxy" or "-COOH" (that is, -CO₂R^{a} where R^{a} is H) includes the deprotonated forms of those groups, i.e. -O⁻ and -COO⁻ respectively.

In a preferred embodiment, the species of formula (Fl-A) has electron-withdrawing groups at certain positions on the heterocyclic skeleton and not at other positions. In a preferred embodiment, therefore, the fluorophore comprises a species of formula (Fl-B)

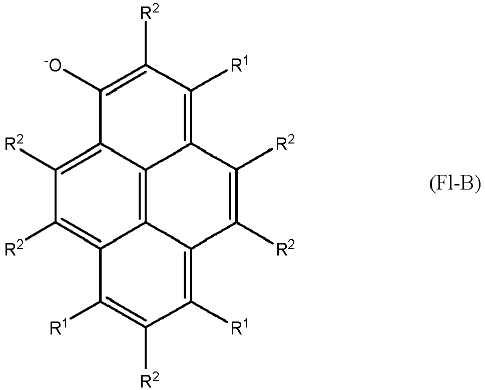

Each R¹ group is independently selected from halogen, cyano, nitro, hydroxy, -OR^{a}, - SO₂R^{a}, -SO₃⁻, -SOR^{a}, -SO₂NR^{a}, -S(O)(NR^{a})R^{a}, -NR^{a}₂, -NR^{a}COR^{a}, -NR^{a}CO₂R^{a}, -COR^{a}, - CO₂R^{a}, -CONR^{a}₂ and a covalent bond to the polymer linking moiety. Usually each R¹ group is independently selected from halogen, cyano, nitro, hydroxy, -OR^{a}, -SO₂R^{a}, -SO₃⁻, -SOR^{a}, - SO₂NR^{a}, -S(O)(NR^{a})R^{a}, -NR^{a}₂, -NR^{a}COR^{a}, -NR^{a}CO₂R^{a}, -COR^{a}, -CO₂R^{a}, and -CONR^{a}2. Preferably each R¹ group is independently selected from hydroxy, -OR^{a}, -SO₂R^{a}, -SO₃⁻, - SOR^{a}, -COR^{a}, -CO₂R^{a}, and -CONR^{a}₂. Particularly preferred examples of R¹ are -OR^{a}, and - SO₃⁻.

Each R² group is independently selected from H, C₁₋₁₀ alkyl, C₃₋₁₀ cycloalkyl, C₄₋₁₀ cycloalkenyl, aryl, alkylaryl, heteroaryl, heterocycloalkyl, C₂₋₁₀ alkenyl, C₂₋₁₀ alkynyl, and a covalent bond to the polymer linking moiety. Usually, each R² group is independently selected from H, C₁₋₁₀ alkyl, C₃₋₆ cycloalkyl, C₄₋₆ cycloalkenyl, and C₂₋₁₀ alkenyl. Preferably, each R² group is independently selected from Hand C₁₋₆ alkyl. Most preferably each R² group is H.

Any R¹ or R² group capable of substitution may be optionally substituted by one or more substituents. The one or more substituents may be present at any suitable position on the R¹ or R² group, including (where present) on an R^{a} group. The one or more substituents are typically selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -OR^{b}, -NR^{b}₂, -COR^{b}, -CO₂R^{b}, and -CONR^{b}₂. Each R¹ or R² is usually substituted by 0, 1, 2 or 3 substituents, preferably by 0 or 1 substituents. Preferred substituents include halogen, oxo, hydroxy, C₁₋₆ alkyl, C₃₋₆ cycloalkyl, C₂₋₆ alkenyl, -OR^{b}, -NR^{b}₂, -COR^{b}, and -CO₂R^{b}. Particularly preferred substituents include halogen, hydroxy, C₁₋₄ alkyl, -OR^{b}, -NR^{b}₂, -COR^{b}, and -CO₂R^{b}.

Where an R¹ or R² group, or a substituent thereon, comprises a hydroxy group or a - COOH group, reference to "hydroxy" or "-COOH" includes the deprotonated forms of those groups, i.e. -O⁻ and -COO⁻ respectively.

R^{a} and R^{b} are as described above.

In a preferred embodiment, each R¹ is independently selected from hydroxy, -OR^{a}, - SO₂R^{a}, -SO₃⁻, -SOR^{a}, -COR^{a}, -CO₂R^{a}, and -CONR^{a}₂; and each R² is independently selected from Hand C₁₋₆ alkyl. R^{a} is independently selected from H, C₁₋₁₀ alkyl, C₃₋₆ cycloalkyl, C₄₋₆ cycloalkenyl, and C₂₋₁₀ alkenyl. In this embodiment, each R¹ and R² group may be unsubstituted or substituted by one substituent independently selected from halogen, hydroxy, C₁₋₄ alkyl, -OR^{b}, -NR^{b}₂, -COR^{b}, and -CO₂R^{b} wherein R^{b} is selected from Hand C₁₋₄ alkyl.

In some embodiments, the fluorophore is fluorescein or a derivative thereof. Fluorescein is a compound having the following structure:

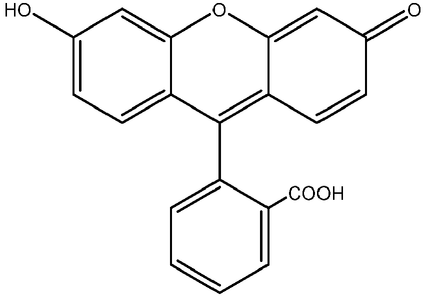

However, structural variations on fluorescein are known to act as fluorophores. Thus, where the fluorophore is a derivative of fluorescein or is based on fluorescein, the fluorophore may for instance comprise a species of formula (Fl-C)

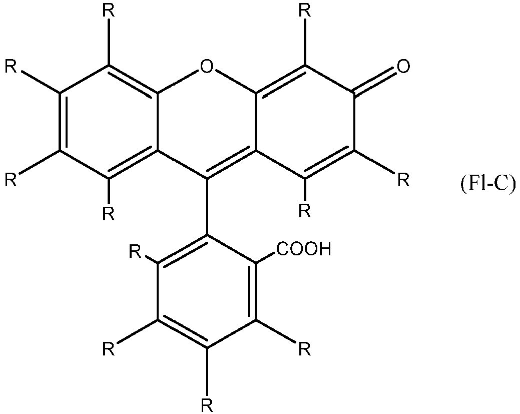

Wherein R is as defined above and may be optionally substituted as described above. For instance, the fluorophore may comprise a species of formula (Fl-D)

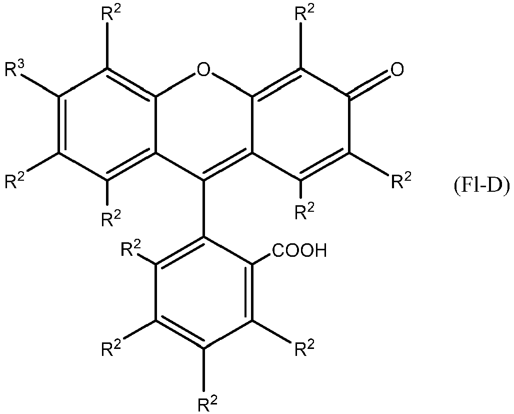

R² is as defined above.

R³ is selected from cyano, nitro, hydroxy, -OR^{a}, -SO₂R^{a}, -SO₃⁻, -SOR^{a}, -SO₂NR^{a}, - S(O)(NR^{a})R^{a}, -NR^{a}₂, -NR^{a}COR^{a}, -NR^{a}CO₂R^{a}, -COR^{a}, -CO₂R^{a}, -CONR^{a}₂ and a covalent bond to the polymer linking moiety. Usually, R³ is selected from hydroxy, -NR^{a}₂, -NR^{a}COR^{a}, and -COOH. Preferably, R³ is selected from hydroxy, -NR^{a}₂, and -CO₂R^{a}; especially hydroxy.

Any R² or R³ group which is capable of substitution may be optionally substituted by one or more substituents. The one or more substituents may be present at any suitable position on the R² or R³ group, including (where present) on an R^{a} group. The one or more substituents are typically selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -OR^{b}, -NR^{b}₂, -COR^{b}, -CO₂R^{b}, and -CONR^{b}₂. Each R² or R³ is usually substituted by 0, 1, 2 or 3 substituents, preferably by 0 or 1 substituents. Preferred substituents include halogen, oxo, hydroxy, C₁₋₆ alkyl, C₃₋₆ cycloalkyl, C₂₋₆ alkenyl, -OR^{b}, -NR^{b}₂, -COR^{b}, and -CO₂R^{b}. Particularly preferred substituents include halogen, hydroxy, C₁₋₄ alkyl, -OR^{b}, -NR^{b}₂, -COR^{b}, and -CO₂R^{b}.

R^{a} and R^{b} are as described above.

In a preferred embodiment, each R² is independently selected from H and C₁₋₆ alkyl, R³ is selected from hydroxy, -NR^{a}₂, and -CO₂R^{a}, and each R^{a} is independently selected from H, C₁₋₁₀ alkyl, C₃₋₆ cycloalkyl, C₄₋₆ cycloalkenyl, and C₂₋₁₀ alkenyl. In this embodiment, each R² and R³ group may be unsubstituted or substituted by one substituent independently selected from halogen, hydroxy, C₁₋₄ alkyl, -OR^{b}, -NR^{b}₂, -COR^{b}, and -CO₂R^{b} wherein R^{b} is selected from Hand C₁₋₄ alkyl.

As with R¹ and R², where an R³ group, or a substituent thereon, comprises a hydroxy group or a -COOH group, reference to "hydroxy" or "-COOH" (that is, -CO₂R^{a} where R^{a} is H) includes the deprotonated forms of those groups, i.e. -O⁻ and -COO⁻ respectively.

### Counter-ion moiety

A key function of the counter-ion moiety is to form an ion pair with the deprotonated form of the fluorophore present at near-neutral pH. Thus, the counter-ion moiety carries a positive charge at near-neutral pH. Of course, the counter-ion moiety may be negatively charged at more extreme pH values.

In addition to carrying a positive charge at near-neutral pH, the counter-ion moiety is in general stable in aqueous solution. Many suitable water-stable positively-charged cations are known to the skilled person.

The counter-ion moiety may be suspended in the polymer matrix of the invention or may be covalently bound to the polymeric support by a polymer linking moiety. It is in general preferred that the counter-ion moiety is covalently bound to the polymeric support. This conveniently allows a large amount of the counter-ion moiety to be present, meaning that an excess can be provided compared to the quantity of fluorophore present, with no limitation due to the solubility of the counter-ion. A covalent bond from the polymeric support to the counter-ion moiety is easily formed where the counter-ion moiety comprises an organic moiety. Thus, in a preferred embodiment, the counter-ion moiety comprises an organic moiety.

Suitable organic cations include quaternary ammonium ions. Thus, in a preferred embodiment, the counter-ion moiety comprises a quaternary ammonium ion. For example, the counter-ion moiety may comprise a quaternary ammonium cation of formula (CI-A):

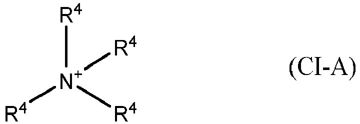

Each R⁴ group is independently selected from H; or C₁₋₃₀ alkyl, C₃₋₁₂ cycloalkyl, C₄₋₁₂ cycloalkenyl, aryl, alkylaryl, heteroaryl, heterocycloalkyl, C₂₋₃₀ alkenyl, C₂₋₃₀ alkynyl, and a covalent bond to the polymer linking moiety; or two R⁴ groups may be joined together to form a C₃₋₁₆ heterocycloalkyl or a C₄₋₁₆ heterocycloalkenyl group. Preferably, each R⁴ group is independently selected from H, C₁₋₂₀ alkyl, C₃₋₆ cycloalkyl, C₂₋₂₀ alkenyl, and a covalent bond to the polymer linking moiety. Particular examples of R⁴ are H, C₁₋₂₀ alkyl, C₂₋₂₀ alkenyl and a covalent bond to the polymer linking moiety.

In a typical embodiment, one R⁴ group is a covalent bond to the polymer linking moiety and the remaining three R⁴ groups are each independently selected from H, C₁₋₂₀ alkyl, C₃₋₁₀ cycloalkyl, C₄₋₁₂ cycloalkenyl, aryl, alkylaryl heteroaryl, heterocycloalkyl, C₂₋₂₀ alkenyl, C₂₋₂₀ alkynyl. Preferably, one R⁴ group is a covalent bond to the polymer linking moiety and the remaining three R⁴ groups are each independently selected from H, C₁₋₂₀ alkyl, and C₂₋₂₀ alkenyl.

Any R⁴ group capable of substitution may be optionally substituted by one or more substituents. The one or more substituents are typically selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, - OR^{b}, -NR^{b}₂, -COR^{b}, and -CO₂R^{b}. Each R⁴ is usually substituted by 0, 1, 2 or 3 substituents, preferably by 0 or 1 substituents. Preferred substituents include halogen, oxo, hydroxy, C₁₋₆ alkyl, C₃₋₆ cycloalkyl, C₂₋₆ alkenyl, -OR^{b}, -NR^{b}₂, -COR^{b}, and -CO₂R^{b}. Particularly preferred substituents include halogen, hydroxy, C₁₋₄ alkyl, -OR^{b}, -NR^{b}₂, -COR^{b}, and -CO₂R^{b}.

R^{b} is as defined above.

In a preferred embodiment, one R⁴ group is a covalent bond to the polymer linking moiety and the remaining three R⁴ groups are each independently selected from H, C₁₋₂₀ alkyl, and C₂₋₂₀ alkenyl. Each R⁴ group capable of substitution is unsubstituted or substituted by one substituent independently selected from halogen, hydroxy, C₁₋₄ alkyl, -OR^{b}, -NR^{b}₂, - COR^{b}, and -CO₂R^{b} wherein R^{b} is selected from Hand C₁₋₄ alkyl.

In some embodiments, the counter-ion moiety comprises a hexadecyltrimethylammonium ion or a derivative thereof. For instance, the counter-ion moiety may comprise (C₁₆H₃₃)(CH₃)₃N⁺; or (C₁₆H₃₃)(CH₃)₂R⁴N⁺, wherein R⁴ is a covalent bond to the polymer linking moiety.

The counter-ion moiety may be associated with one or more negatively-charged ions other than the negatively-charged fluorophore. These negatively-charged ions are not covalently bound to the counter-ion moiety or the polymer matrix. Such ions are dissociated from the counter-ion moiety when the counter-ion moiety and associated ion(s) are contacted with water. Suitable negative ions include halide ions or hydroxide ions; preferably hydroxide ions. The associated ion may typically be present where the positively-charged counter-ion moiety is introduced to the polymer matrix by suspending a dissolved salt containing the counter-ion moiety and one or more associated negative ions in the polymer matrix.

### Polymer linking moiety

The polymer matrix comprises a polymer linking moiety which forms a covalent attachment between the polymeric support and either the fluorophore or the counter-ion moiety. In some embodiments, the polymer linking moiety is a covalent bond directly joining the polymeric support to the fluorophore or counter-ion moiety. In other embodiments, the polymer linking moiety may comprise one or more intervening atoms.

Preferably, the linker is stable to hydrolysis during storage. For instance, it is preferred that the linker is stable with respect to hydrolysis during storage for a period of up to 1, 2, 3, 4, 5 or 6 months. By "stable to hydrolysis" is mean that at least 90%, preferably at least 95%, of the linker does not hydrolyse.

The nature of the polymer linking moiety is not particularly limited. The polymer linking moiety may include one or more of a covalent bond, O, S, N, and C. Typically, the polymer linking moiety comprises one or more of a covalent bond, -O-, -S-, -SO₂-, -SO-, an optionally substituted alkylene, an optionally substituted alkenylene, an optionally substituted alkynylene, and -NR⁵-. For example, the polymer linking moiety may comprise one or more of a covalent bond, -O-, -NR⁵-, -S-, -SO₂-, -SO-, an optionally substituted C₁₋₁₀ alkylene, and an optionally substituted C₂₋₁₀ alkenylene.

Where the polymer linking moiety comprises more than one of the aforementioned divalent species, the polymer linking moiety typically comprises two or three moieties independently selected from -O-, -NR⁵-, -S-, -SO₂-, -SO-, an optionally substituted C₁₋₁₀ alkylene, and an optionally substituted C₂₋₁₀ alkenylene. At least two of the moieties differ.

Preferably, the polymer linking moiety comprises one or more moieties independently selected from a covalent bond, -O-, -NR⁵-, and an optionally substituted C₁₋₆ alkylene. Examples of a polymer linking moiety include a covalent bond, -O-, -NH-, -NR⁵-, and C₁₋₄ alkylene.

R⁵ is typically selected from H, C₁₋₁₀ alkyl, C₃₋₁₀ cycloalkyl, C₄₋₁₀ cycloalkenyl, aryl, arylalkyl, heteroaryl, heterocycloalkyl, C₂₋₁₀ alkenyl, C₂₋₁₀ alkynyl, -COR^{a}, -CO₂R^{a}, and - CONR^{a}₂. Preferably, R⁵ is selected from H, C₁₋₆ alkyl, C₃₋₆ cycloalkyl, and C₂₋₁₀ alkenyl. Particularly preferably, R⁵ is selected from Hand C₁₋₄ alkyl. Examples of R⁵ include hydrogen, methyl and ethyl groups.

The polymer linking moiety may optionally be substituted by one or more substituents. The one or more substituents may be present at any suitable position on the polymer linking moiety, including (where present) on an R^{a} group. In particular, an alkylene, alkenylene or alkynylene moiety of the polymer linking moiety may be optionally substituted. The one or more substituents are typically selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, - OR^{b}, -NR^{b}₂, -COR^{b}, -CO₂R^{b}, and -CONR^{b}₂. The polymer linking moiety is usually substituted by 0, 1, 2 or 3 substituents, preferably by 0 or 1 substituents. Preferred substituents include halogen, oxo, hydroxy, C₁₋₆ alkyl, C₃₋₆ cycloalkyl, C₂₋₆ alkenyl, -OR^{b}, - NR^{b}₂, -COR^{b}, and -CO₂R^{b}. Particularly preferred substituents include halogen, hydroxy, C₁₋₄ alkyl, -OR^{b}, -NR^{b}₂, -COR^{b}, and -CO₂R^{b}.

Preferably the polymer linking moiety is unsubstituted
R^{a} and R^{b} are as described above.

In a preferred embodiment, the polymer linking moiety comprises one, two or three moieties independently selected from a covalent bond, -O-, -NR⁵-, and an optionally substituted C₁₋₆ alkylene. R⁵ is selected from Hand C₁₋₄ alkyl. In this embodiment, the polymer linking moiety may be unsubstituted or substituted by one substituent independently selected from halogen, hydroxy, C₁₋₄ alkyl, -OR^{b}, -NR^{b}₂, -COR^{b}, and -CO₂R^{b} wherein R^{b} is selected from Hand C₁₋₄ alkyl. Particularly preferred examples of a polymer linking moiety include a covalent bond, -O- and -CH₂-.

For completeness, it is noted that where any of R or R¹ to R³ is a covalent bond attaching the fluorophore to the polymer linking moiety and the polymer linking moiety is a covalent bond, the fluorophore is bound to the polymeric support by a covalent bond at the respective R or R¹ to R³ position. Similarly, where R⁴ is a covalent bond attaching the counter-ion to the polymer linking moiety and the polymer linking moiety is a covalent bond, the counter-ion moiety is bound to the polymeric support by a covalent bond at the relevant R⁴ position.

### Polymeric support

The polymer matrix comprises a polymeric support. A polymeric support is a polymer which may comprise structural units derived from one type of monomer or a plurality of different monomers. In general, at least the majority of the structural units present in the polymeric support are derived from one type of monomer.

The polymeric support is generally gas-permeable. Where the polymer matrix is gas-permeable, a gas such as CO₂ may diffuse into the matrix and the polymer matrix may be used as a gas sensor. The polymeric support may further be permeable to liquid, such as water.

In some embodiments, which are preferred, the polymeric support comprises a hydrophilic polymer. For instance, the polymeric support may comprise or consist of structural units derived from polymerisation of a hydrophilic monomer. Where the polymeric support comprises structural units derived from polymerisation of a hydrophilic monomer, the polymeric support is itself typically hydrophilic.

Suitable examples of a hydrophilic polymer which may be comprised in the polymeric support include a hydrogel, a cellulose derivative, ethyl cellulose, a sol-gel, or a hydrophilic silicone-based polymer. Preferably, the polymeric support comprises a hydrogel. Suitable examples of a hydrogel include polyacrylamide or polyhydroxyethyl methacrylate.

In some embodiments, the polymeric support comprises a hydrophobic polymer. For instance, the polymeric support may comprise or consist of structural units derived from polymerisation of a hydrophobic monomer. Where the polymeric support comprises structural units derived from polymerisation of a hydrophobic polymer, the polymeric support is itself typically hydrophobic.

For example, the polymeric support may comprise one or more of polystyrene, a hydrophobic silicone-based polymer, a hydrophobic cellulose derivative, or plasticised ethyl cellulose. Preferred among these is polystyrene.

The polymeric support may comprise structural units which include either the fluorophore or the counter-ion moiety. In a preferred embodiment, the polymeric support comprises structural units which include the counter-ion moiety. Inclusion of such structural units in the polymer matrix may be achieved in various ways, as follows.
(i) The polymeric support may be produced by co-polymerising (a) a monomer comprising the fluorophore covalently bound to a polymerisable moiety by a polymer linking moiety and (b) one or more other monomers.
(ii) The polymeric support may be produced by co-polymerising (a) a monomer comprising the counter-ion moiety covalently bound to a polymerisable moiety by a polymer linking moiety and (b) one or more other monomers.
(iii) The polymeric support may be produced by co-polymerising (a) a monomer comprising a polymer linking moiety precursor and a polymerisable moiety and (b) one or more other monomers. A "polymer linking moiety precursor" is a species which can be reacted with a fluorophore precursor or a counter-ion moiety precursor to form a fluorophore or a counter-ion moiety covalently bound to the polymeric support via the polymer linking moiety. The polymer linking moiety precursor is therefore reacted with a fluorophore precursor or a counter-ion moiety precursor to produce a fluorophore or a counter-ion moiety covalently bound to the polymeric support via the polymer linking moiety.

### Polymer matrix

The polymer matrix comprises a fluorophore, a positively charged counter-ion moiety and a polymeric support. However, when the polymer matrix is used in optical sensing as described herein, the polymer matrix must comprise water to mediate the transfer of H⁺ to and from the fluorophore. Accordingly, in some embodiments, the polymer matrix comprises water. Typically, the molar ratio of water (H₂O) to fluorophore is 1:1 or greater. This ensures that enough H₂O is present to interact with each ion pair. For example, the molar ratio of water (H₂O) to fluorophore may be at least 2:1 or at least 5:1.

Preferably, the polymer matrix comprises a known quantity of water.

The molar quantity of water present is typically in excess compared to the molar quantity of fluorophore present. The molar quantity of water present may also be in excess compared to the molar quantity of counter-ion moiety.

In some embodiments, the polymer matrix is saturated with water.

### Optical sensor

The polymer matrix is useful in an optical sensor for optical sensing of CO₂. An optical sensor generates an optical signal which varies with pH, in the presence of CO₂. The optical signal can be generated by directing excitation light onto the polymer matrix while the polymer matrix contacts a sample (directly or indirectly). Accordingly, described herein is an optical sensor 1 for optical sensing of CO₂, comprising:
- a sensing region comprising a polymer matrix 5 as described herein; and
- an optical waveguide 3 arranged to direct light onto the sensing region.

An optical waveguide is a physical structure having a first end and a second end, capable of guiding electromagnetic waves in the optical region of the spectrum between the first and second ends of the structure.

The optical sensor 1 is suitable for detecting the CO₂ content of a sample 9. CO₂ present in a sample is referred to herein as an analyte. The sample 9 may be any fluid, the CO₂ content of which is of interest. Exemplary samples include buffers, and biological samples such as saliva or blood. In a preferred embodiment, the sample 9 is a blood sample, for instance a blood sample taken from a human patient. Thus, in a preferred embodiment, the optical is a sensor for detecting the CO₂ content of blood.

Where the sample 9 is a biological sample, the sample 9 is typically an *ex vivo* sample; that is, the sample 9 is typically outside the human or animal body.

The design of an optical sensor 1 comprising the polymer matrix 5 of the invention may be influenced by the nature of the polymeric support, and in particular whether the polymeric support is hydrophobic or hydrophilic.

Where the polymeric support is hydrophobic in nature, the polymer matrix 5 may be used to construct a so-called "naked sensor". Where the polymeric support is hydrophobic, the polymer matrix 5 can be placed in direct contact with an aqueous solution (for instance a biological sample, such as blood), and the aqueous solution will not penetrate the polymer matrix 5, or will penetrate the polymer matrix 5 to a small extent. Further, where the polymeric support is hydrophobic, it will advantageously resist adsorption of hydrophilic proteins. In such cases, it is not necessary to provide an additional layer to prevent penetration of the aqueous solution or proteins into the polymer matrix 5.

A naked sensor (which does not comprise a membrane arranged to prevent penetration of an aqueous sample into the polymer matrix 5) has various advantages. A membrane can slow the passage of analyte and can act as a reservoir for analyte; this increases the response time of the sensor. Naked sensors therefore have exceptionally rapid response times.

There are also especial advantages to optical sensors constructed using a hydrophilic polymer matrix. In use as an optical sensor, the polymer matrix 5 must contain water (to enable the formation of carbonic acid during the detection of CO₂). If the water content is too low, not all CO₂ may be able to generate carbonic acid. Thus, not all CO₂ present is available to cause protonation of the fluorophore and hence to alter the absorption or emission characteristics of the polymer matrix 5. Thus, insufficient water content may lead to an optical sensor whose optical output does not accurately correspond to the CO₂ content of the sample 9 under test.

Where a hydrophobic polymer matrix 5 is used, it is difficult to ensure that the polymer matrix 5 contains enough water to enable the sensing chemistry. It is therefore convenient to use a hydrophilic polymer matrix 5 in the optical sensor 1 as it can readily become saturated with water. Where a hydrophilic polymer matrix 5 is contacted with water, it will take up and hold onto a specific amount of water. A hydrophilic polymer matrix 5 (e.g. a hydrogel) may be saturated with water.

If a hydrophilic polymer matrix 5 is placed into direct contact with an aqueous solution (for instance a biological sample, such as blood), the aqueous solution will penetrate the hydrophilic polymer matrix 5. Accordingly, where the optical sensor 1 comprises a hydrophilic polymer matrix 5, the optical sensor 1 typically also comprises a hydrophobic membrane 7 arranged such that the sample 9 contacts the polymer matrix 5 through the hydrophobic membrane 7. Thus, the optical sensor 1 comprising a hydrophilic polymer matrix 5 may comprise a hydrophobic membrane 7 arranged to allow analyte to enter the polymer matrix 5 through the hydrophobic membrane 7.

Where an optical sensor comprises a hydrophilic polymer matrix 5 and a gas-permeable hydrophobic membrane 7, and the optical sensor is placed in contact with an aqueous solution, gaseous water (water vapour) may pass across the gas permeable hydrophobic membrane 7. However, equilibrium is maintained and so the quantity of water present in the polymer matrix 5 remains approximately constant. Accordingly, an optical sensor comprising a hydrophilic polymer matrix 5 is less sensitive to inaccuracies arising from the presence of insufficient water, or varying water content.

In some embodiments, therefore, the optical sensor 1 comprises a membrane 7. Generally, the membrane 7 is gas-permeable. The membrane 7 may further be liquid-permeable. Typically, where the optical sensor 1 comprises a membrane 7, the optical sensor 1 is configured to allow an analyte to enter the sensing region through the membrane 7. In such embodiments the membrane 7 is typically hydrophobic and, preferably, the polymer matrix 5 is hydrophilic.

In some embodiments, the polymer matrix 5 may penetrate the membrane 7. The membrane 7 may be disposed within or partially within the polymer matrix 5.

Suitable membranes, particularly gas-permeable membranes, are known in the art. Such membranes include dialysis membranes and microporous membranes; dialysis membranes are preferred.

The optical sensor 1 is configured specifically for the detection of CO₂. In a particularly preferred embodiment, there is provided an optical sensor 1 for optical sensing of CO₂, comprising:
- a sensing region comprising a polymer matrix 5 as described herein, wherein the sensing region further comprises a hydrophobic membrane 7, and the sensor is configured to allow an analyte to enter the sensing region through the hydrophobic membrane 7; and
- an optical waveguide 3 arranged to direct light onto the sensing region.

The analyte is CO₂.

In use, the hydrophobic membrane 7 is disposed between the sample 9 under test and the sensing region. The hydrophobic membrane 7 therefore acts as a barrier to liquids and can prevent liquids (particularly water) in the sample 9 under test from entering the sensing region. Preferably, therefore, the hydrophobic membrane 7 is impermeable to liquids. This prevents any acid in the sample 9 from entering the sensor. However, gaseous or dissolved CO₂ in the sample 9 under test can diffuse across the hydrophobic membrane 7 into the sensing region, as the hydrophobic membrane 7 is gas-permeable. Thus, where the sensor comprises a hydrophobic membrane 7, the optical sensor 1 specifically senses CO₂. Moreover, the sensor does not suffer from interference from species contained in a liquid sample 9 such as acids or alkalis.

Particularly preferably, the hydrophobic membrane 7 is impermeable to hydrogen ions.

In this CO₂-specific configuration, it is preferred that the polymer matrix 5 is hydrophilic, for the reasons explained above. The hydrophilic polymer matrix 5 can easily trap the water needed to enable the formation of carbonic acid and assist the functioning of the sensor.

Thus, in a particularly preferred embodiment, the optical sensor 1 is an optical sensor 1 for optical sensing of CO₂, comprising:
- a sensing region comprising a polymer matrix 5 as described herein, wherein the polymeric support comprises a hydrophilic polymer; and the sensing region further comprises a gas-permeable hydrophobic membrane 7 which is impermeable to liquids and hydrogen ions; and the sensor is configured to allow CO₂ to enter the sensing region through the hydrophobic membrane 7; and
- an optical waveguide 3 arranged to direct light onto the sensing region.

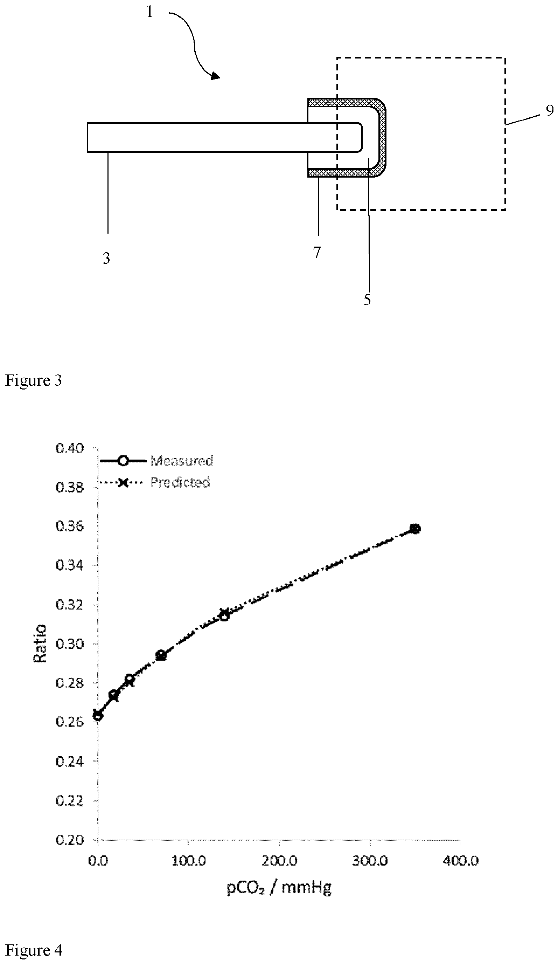

A diagram of an optical sensor 1 in use is shown in Figure 3; in the embodiment shown, the optical sensor 1 comprises a gas-permeable membrane 7 but this is not necessary. The optical waveguide 3 is arranged to direct excitation light onto a sensing region which includes the polymer matrix 5. In use, the sensing region contacts a sample 9. A gas-permeable membrane 7 is disposed on the polymer matrix 5 and configured such that an analyte (acid or CO₂) present in the sample 9 may pass through the membrane 7 (if present) in order to contact the polymer matrix 5. The fluorophore will absorb the excitation light and may emit light; light emitted by the fluorophore passes through the optical waveguide 3. The absorption and emission spectra will depend on the pH of the sample 9, which determines the extent of protonation of the fluorophore.

The waveguide may be any optically transmissive material. Typically, the waveguide comprises or consists of an optical fibre; alternatively, a transparent optical window may be used. Optical fibres use total internal reflection to prevent light being lost from the fibre. This means light can be efficiently carried to and from the fluorophore, improving the signal and providing for higher-quality and more reliable measurements.

Optionally, the optical sensor 1 may comprise a reflector configured to reflect light emitted by the fluorophore into the optical waveguide 3. The reflector, where present, increases the proportion of light emitted by the fluorophore which can be collected by the waveguide and subsequently detected. The reflector may comprise a layer deposited over the polymer matrix 5, for instance over a membrane 7 disposed on the polymer matrix 5. Suitable materials which may be used as reflectors include polysulfones (PSU), polyethersulfones (PESU), and polyphenylsulfones (PPSU). Polysulfones are preferred. It would also be possible to use other reflecting compounds such as silicon containing titanium oxide, or barium sulfate.

The optical sensor 1 comprising a sensing region and an optical waveguide 3, and optionally also comprising membrane 7 and/or a reflector may be referred to as a sensor probe.

The optical sensor 1 may further comprise a light source configured to provide excitation light to the fluorophore. The light source may be any light source capable of emitting light at the wavelengths and intensities required to excite the fluorophore. For example, the light source may comprise a laser diode.

The optical sensor 1 may further comprise a detector configured to detect light emitted by the fluorophore through the optical waveguide 3. The detector may be any device capable of producing a signal in response to receiving light at the wavelengths emitted by the fluorophore. For example, the detector may comprise a charge-coupled device, an active-pixel sensor, a photodiode, or photoresistor.

Some or all of the optical sensor 1 may be disposable. This is convenient in clinical contexts, where optical sensor 1 may be contacted with a biological sample inside or taken from a patient. In such cases, the part of the optical sensor 1 which contacts the biological sample should be sterile and cannot be reused between patients. For example, the sensor probe may be disposable and not the detector or light source if present. In such a case, the optical sensor 1 may further comprise a connector interface configured to connect the optical waveguide 3 to the light source and the detector.

The optical sensor 1 may form part of an optical sensing system which further comprises a control system. The control system may be configured to cause the light source to emit light, and optionally to activate the detector if necessary. The optical sensing system may further comprise an analysis system. The analysis system may be configured to determine whether an analyte (carbonic acid) is present in the sample 9 under test. In particular, the analysis system may be configured to determine the CO₂ concentration of the sample 9 under test.

The optical sensor 1 and optical sensing system described herein can be used to provide rapid, real-time measurements of the CO₂ content of a sample 9. This is particularly desirable in blood gas sensing. It is important to monitor the CO₂ content of a patient's blood during procedures such as dialysis or surgery, particularly heart bypass surgery, as CO₂ content can vary rapidly with dangerous consequences for the patient.

Accordingly, the optical sensing system may be an in-line blood gas sensing system comprising an optical sensor 1 as described herein, wherein the in-line blood gas sensing system is configured to direct blood from the body of the patient outside the body and into contact with the sensing region of the optical sensor 1. Usually, the in-line blood gas sensing system is further configured to return the blood back into the body of the patient.

Generally, the in-line blood gas sensing system additionally comprises an optical sensor 1 as described herein, a blood line, and a pump, wherein the blood line is configured to direct blood from the body of the patient outside the body and into contact with the sensing region of the optical sensor 1; and to return the blood into the body of the patient. The pump is configured to pump blood along the blood line.

In one example, the disclosure relates to a dialysis system comprising an in-line blood gas sensing system as described herein. In another example, the invention relates to a cardiac bypass system comprising an in-line blood gas sensing system as described herein.

### Method for producing a polymer matrix

The polymer matrix of the invention may be produced by any suitable method. For instance, it would be possible to generate a polymer matrix wherein the fluorophore or the counter-ion moiety is suspended within the polymer matrix already, by performing a polymerisation reaction to produce a polymeric support in the presence of the fluorophore or counter-ion moiety. However, it is convenient to first generate the polymeric support having a fluorophore or counter-ion moiety covalently bound thereto, and then to provide the fluorophore or counter-ion moiety (whichever is not bound to the polymeric support) within the matrix. This method allows any waste products or leftover starting materials from the generation of the polymeric support to be washed away.

Thus, described herein is a method of producing a polymer matrix for optical sensing, the method comprising:
(i) providing a polymeric support, wherein either a fluorophore or a counter-ion moiety is covalently bound to the polymeric support by a polymer linking moiety; and
(ii) suspending either a fluorophore or a counter-ion moiety, whichever is not covalently bound to the polymeric support via the polymer linking moiety, in the polymeric support.

Generally, the polymer matrix for optical sensing produced by the disclosed method is a polymer matrix as described herein.

Step (i) may comprise covalently attaching a fluorophore or a counter-ion moiety to a polymeric support. For instance, step (i) may comprise:
- generating a polymeric support by polymerising a monomer; and
- reacting the polymeric support with a fluorophore precursor comprising a fluorophore, or with a counter-ion precursor comprising a counter-ion moiety, to generate a fluorophore or a counter-ion moiety covalently bound to the polymeric support by a polymer linking moiety.

A suitable monomer is any polymerisable moiety, typically a hydrophobic polymerisable moiety or a hydrophilic polymerisable moiety. Examples include styrene or hydrogel monomers.

In another example, step (i) may comprise:
- functionalising a polymeric support; and
- reacting the functionalised polymeric support with a fluorophore precursor comprising a fluorophore, or with a counter-ion precursor comprising a counter-ion moiety, to generate a fluorophore or a counter-ion moiety covalently bound to the polymeric support by a polymer linking moiety.

This method may be useful where an existing polymer is desired as the polymeric support, for example cellulose or a derivative thereof.

Conveniently, the polymeric support may be directly produced incorporating the fluorophore or counter-ion moiety. In such an embodiment, step (i) comprises co-polymerising a fluorophore monomer or a counter-ion monomer with one or more other monomers. Preferably, step (i) comprising co-polymerising a counter-ion monomer with one or more other monomers.

By a "fluorophore monomer" is meant a polymerisable monomer comprising the fluorophore. A suitable "fluorophore monomer" is a fluorophore as described herein wherein the covalent bond attaching the fluorophore to a polymer linking moiety is replaced by a polymerisable moiety, typically an alkenyl or alkynyl group.

By a "counter-ion monomer" is meant a polymerisable monomer comprising the counter-ion moiety. A suitable "counter-ion monomer" is a counter-ion moiety as described herein wherein the covalent bond attaching the counter-ion moiety to a polymer linking moiety is replaced by a polymerisable moiety, typically an alkenyl or alkynyl group.

An exemplary counter-ion monomer is a quaternary ammonium cation of formula (CI-AM):

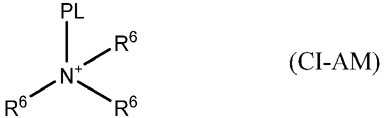

PL is a polymerisable moiety. Preferably, PL is an alkenyl or alkynyl group. For instance, PL may be a C₂₋₁₀ alkenyl group or a C₂₋₁₀ alkynyl group. Particularly preferably, PL is a C₂₋₆ alkenyl group.

Each R⁶ group is independently selected from H; or C₁₋₃₀ alkyl, C₃₋₁₂ cycloalkyl, C₄₋₁₂ cycloalkenyl, aryl, alkylaryl, heteroaryl, heterocycloalkyl, C₂₋₃₀ alkenyl, and C₂₋₃₀ alkynyl; or two R⁸ groups may be joined together to form a C₃₋₁₆ heterocycloalkyl or a C₄₋₁₆ heterocycloalkenyl group. Preferably, each R⁶ group may be independently selected from H, C₁₋₁₀ alkyl, C₃₋₆ cycloalkyl, and C₂₋₁₀ alkenyl. The most preferred examples of R⁶ are H and C₁₋₆ alkyl.

PL and/or any R⁶ group capable of substitution may be optionally substituted by one or more substituents. The one or more substituents may be present at any position capable of substitution. The one or more substituents are typically selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, - OR^{b}, -NR^{b}₂, -COR^{b}, -CO₂R^{b}, and -CONR^{b}₂. PL and R⁶ are usually substituted by 0, 1, 2 or 3 substituents, preferably by 0 or 1 substituents. Preferred substituents include halogen, oxo, hydroxy, C₁₋₆ alkyl, C₃₋₆ cycloalkyl, C₂₋₆ alkenyl, -OR^{b}, -NR^{b}₂, -COR^{b}, and -CO₂R^{b}. Particularly preferred substituents include halogen, hydroxy, C₁₋₄ alkyl, -OR^{b}, -NR^{b}₂, -COR^{b}, and -CO₂R^{b}. Most preferably Pl and R⁶ are unsubstituted.

R^{b} is as described above.

In a preferred embodiment, PL is a C₂₋₆ alkenyl group and each R⁶ group is independently selected from Hand C₁₋₆ alkyl. PL and R⁶ are each unsubstituted or substituted by one substituent independently selected from halogen, hydroxy, C₁₋₄ alkyl, - OR^{b}, -NR^{b}₂, -COR^{b}, and -CO₂R^{b} wherein R^{b} is selected from H and C₁₋₄ alkyl.

Intermediate steps may be performed between step (i) and step (ii). For instance, the polymeric support having a fluorophore or a counter-ion moiety covalently bound thereto may be washed before step (ii) is performed.

Step (ii) involves suspending a fluorophore or a counter-ion moiety in the polymer matrix. For example, step (ii) may involve dissolving the fluorophore (e.g. a salt form of the fluorophore) in a coating solution such as water or an aqueous solution, and contacting the polymeric support produced in step (i) with the coating solution comprising the dissolved fluorophore. Alternatively, step (ii) may involve dissolving the counter-ion moiety (e.g. a salt comprising the counter-ion moiety, typically a hydroxide salt of the counter-ion moiety) in a coating solution such as water or an aqueous solution, and contacting the polymeric support produced in step (i) with the coating solution comprising the dissolved counter-ion moiety.

Further steps may be performed after step (ii). For instance, the method of producing a polymer matrix may further comprise drying the polymer matrix to remove excess water.

### Method of producing an optical sensor

The polymer matrix may be used to provide an optical sensor. Thus, described herein is a method of producing an optical sensor, the method comprising:
(i) providing an optical waveguide;
(ii) disposing a polymer matrix as described herein on the optical waveguide; and
(iii)optionally disposing a membrane on the polymer matrix.

Generally, the optical sensor produced by this method is an optical sensor 1 as described herein. For example, the membrane is typically a gas-permeable membrane and preferably also hydrophobic.

Disposing a polymer matrix onto the optical waveguide does not always require the polymer matrix to be in direct contact with the optical waveguide. There may, for instance, be an intervening coating present on the optical waveguide.

Step (ii) may in some cases include providing a polymer matrix as described herein, comprising both a fluorophore and a counter-ion moiety (one of which is covalently attached to the polymeric support) and then disposing that polymer matrix on the optical waveguide.

The method of providing the polymer matrix may be as described herein. For instance, the method of producing an optical sensor may comprise:
(i) providing an optical waveguide;
(ii) providing a polymeric support, wherein either a fluorophore or a counter-ion moiety is covalently bound to the polymeric support by a polymer linking moiety, and suspending either a fluorophore or a counter-ion moiety, whichever is not covalently bound to the polymeric support via the polymer linking moiety, in the polymeric support, to produce a polymer matrix as described herein; and
disposing the polymer matrix on the optical waveguide.

Alternatively, step (ii) may involve generating a polymer matrix as described herein *in situ,* on the optical waveguide. The method of generating the polymer matrix may be as described herein. For instance, the method of producing an optical sensor may comprise:
(i) providing an optical waveguide;
(ii)
   (a) disposing a polymeric support on the optical waveguide, wherein either a fluorophore or a counter-ion moiety is covalently bound to the polymeric support by a polymer linking moiety; and
   (b) suspending either a fluorophore or a counter-ion moiety, whichever is not covalently bound to the polymeric support via the polymer linking moiety, in the polymeric support.

In a typical example, the method of producing an optical sensor may comprise:
(i) providing an optical waveguide;
(ii)
   (a) contacting the optical waveguide with a solution comprising a fluorophore monomer or a counter-ion monomer with one or more other monomers and initiating polymerisation, to produce a polymeric support disposed on the optical waveguide wherein either a fluorophore or a counter-ion moiety is covalently bound to the polymeric support by a polymer linking moiety; and
   (b) suspending either a fluorophore or a counter-ion moiety, whichever is not covalently bound to the polymeric support via the polymer linking moiety, in the polymeric support, to produce a polymer matrix as described herein.

A washing step may be performed between (ii)(a) and (ii)(b), to remove unreacted monomers and/or waste products.

It may be desirable to incorporate a membrane into the optical sensor during its production. This can be done after the polymer matrix of the invention is generated and is disposed on the sensor. For instance, the method of producing an optical sensor may comprise:
(i) providing an optical waveguide;
(ii) disposing a polymer matrix as described herein on the optical waveguide; and
(iii) subsequently disposing a membrane on the polymer matrix.

In other embodiments, the membrane may be incorporated at an earlier point in the procedure, for instance before the polymer matrix is generated. For example, in some embodiments the method of producing an optical sensor may comprise:
(i) providing an optical waveguide;
(ii)
   (a) contacting the optical waveguide and a membrane with a solution comprising a fluorophore monomer or a counter-ion monomer with one or more other monomers, and initiating polymerisation, to produce a polymeric support disposed on the optical waveguide wherein either a fluorophore or a counter-ion moiety is covalently bound to the polymeric support by a polymer linking moiety; and
   (b) suspending either a fluorophore or a counter-ion moiety, whichever is not covalently bound to the polymeric support via the polymer linking moiety, in the polymeric support.

The solution comprising a fluorophore monomer or a counter-ion monomer with one or more other monomers may penetrate the membrane. For instance, the membrane may be soaked with said solution.

The method of producing an optical sensor may comprise one or more additional steps after (i) and (ii), as follows. These additional steps may be performed in any order.

The method of producing an optical sensor may further comprise a step of contacting the optical sensor with water (in the form of water vapour and/or more preferably liquid water). This allows the water content within the sensor to equilibrate with the external water. This ensures that, when the sensor is contacted with a sample comprising water (for instance an aqueous sample such as an aqueous solution, blood or saliva) the uptake or loss of water by the optical sensor is minimised.

The method of producing an optical sensor may further comprise a step of disposing a reflector on the optical sensor. For instance, the method of producing an optical sensor may comprise a step of disposing a layer comprising one or more of polysulfone (PSU), polyethersulfone (PESU), and polyphenylsulfone (PPSU) on the polymer matrix. Where a gas-permeable membrane is disposed on the polymer matrix, the method of producing an optical sensor may comprise a step of disposing a layer comprising one or more of polysulfone (PSU), polyethersulfone (PESU), and polyphenylsulfone (PPSU) on the gas-permeable membrane. Polysulfones are preferred.

The method of producing an optical sensor may further comprise incorporating a light source and/or a detector. The method may also comprise incorporating a control system and/or an analysis system.

### Method of measurement

The sensor described herein can be used to detect CO₂, in a sample. The optical sensor can further be used to determine the amount of CO₂ in a sample. This can be accomplished by a simple intensity measurement. For instance, the optical output of the optical sensor (usually the light emitted by the fluorophore) may be calibrated using one or more solutions of known CO₂ content. Thus, the intensity I₁ of fluorescent emission at a wavelength λ₁ can be determined as a function of CO₂ content. Alternatively or additionally, the absorbance A₁ at a wavelength λ_{1'} can be determined as a function of CO₂ content. λ₁ and λ_{1'} are the wavelengths at which the protonated form of the fluorophore emits/absorbs light, respectively; a calibration may also be performed for the deprotonated form. The emission (at λ₁) or absorbance (at λ_{1'}) occurring when excitation light is provided to the optical sensor contacting a sample is measured, and can then be used to determine the CO₂ content of the sample.

Although the sensor may be operated in absorption or emission modes, it is preferred to use the sensor to detect fluorescent emission. This is because the path length of light through the sensing region of the optical sensor is generally too small to allow strong absorbance.

Described herein is a method of measuring the CO₂ content of a sample, the method comprising:
(i) contacting an optical sensor as described herein with the sample;
(ii) providing excitation light to the sensing region through the optical waveguide; and
(iii) detecting the intensity I₁ of light emitted from the fluorophore at a first wavelength λ₁ through the optical waveguide.

Contacting the sensor with a sample typically involves flowing the sample over the sensing region of the sensor or dipping the sensing region of the sensor into the sample.

Also described herein is a method of measuring the CO₂ content of a sample using absorption methodology, the method comprising:
(i) contacting an optical sensor as described herein with the sample;
(ii) providing excitation light to the sensing region through the optical waveguide;
(iii) detecting light returning from the sensing region through the optical waveguide; and
(iv) determining the absorbance A₁ of the fluorophore at a first wavelength λ_{1'}.

The method may comprise an initial step of calibrating the sensor.

The method may comprise a subsequent calculation step, involving comparing the detected emission intensity I₁ or absorbance A₁ to a calibration curve in order to determine the CO₂ content of the sample.

A single intensity measurement is advantageously simple. The accuracy of the result may be improved by taking one or more further measurements. Thus, in some embodiments, the method of measuring the CO₂ content of a sample comprises:
(i) contacting an optical sensor as described herein with the sample;
(ii) providing excitation light to the sensing region through the optical waveguide;
(iii) detecting the intensity I₁ of light emitted by the fluorophore at a first wavelength λ₁ through the optical waveguide; and
(iv) detecting the intensity I₂ of light emitted by the fluorophore at a second wavelength λ₂ through the optical waveguide.

This method may further comprise, for instance, determining CO₂ content based on each of I₁ and I₂ and then taking an average (for instance a mean) of the two values in order to arrive at a mean value for the CO₂ content of the sample.

Intensity measurements relying on absolute intensity are subject to inaccuracies occurring when fluorophore moieties are photobleached. Where a fluorophore moiety is photobleached, the total intensity of fluorescence emission decreases, inaccurately suggesting a change in concentration of the analyte. Thus, it is particularly preferred to use a ratiometric measurement to measure the CO₂ content of a sample. A ratiometric measurement method reduces or avoids errors occurring due to photobleaching of the optical sensor between the calibration process and the measurement.

A ratiometric method can be used where the protonated and deprotonated forms of the fluorophore each undergo fluorescent emission at differing wavelengths, and/or where the protonated and deprotonated forms of the fluorophore absorb light at differing wavelengths. The ratiometric method involves comparing the intensity of emission (or absorbance) of the protonated and deprotonated forms, and using the ratio of those two emission intensities (or absorbances) to determine the relative quantities of protonated and deprotonated fluorophore. Thus, the CO₂ content of the sample may be determined.

Determination of the CO₂ content of a sample can be performed either by calibrating the optical sensor (i.e. by determining the output of the sensor when contacted with one or more samples of known CO₂ content). Alternatively, the CO₂ content of a sample can be determined based on a mathematical model of the system.

As with a simple intensity measurement, a ratiometric measurement can be performed in absorption or emission mode. However, it is preferred to perform the measurement in emission mode as the path length of light through the sensing region of the optical sensor is generally too short in practice to allow significant absorption.

Accordingly, described herein is a method of measuring the CO₂ content of a sample, the method comprising:
(i) contacting an optical sensor as described herein with the sample;
(ii) providing excitation light to the sensing region through the optical waveguide;
(iii)detecting the intensity I₁ of light emitted by the fluorophore at a first wavelength λ₁ through the optical waveguide;
(iv)detecting the intensity I₂ of light emitted by the fluorophore at a second wavelength λ₂ through the optical waveguide; and
(v) comparing I₂ to I₁.

The method may comprise an initial step of calibrating the sensor.

The method may comprise a subsequent calculation step, involving comparing the detected emission intensity ration I₁:I₂ to a calibration curve in order to determine the CO₂ content of the sample.

Each optical measurement is performed rapidly, typically taking less than a second. Moreover, the sample volume of the sensing region of the sensor is very small, and typically does not act as a reservoir for the sample. Consequently, the sensor has a very rapid response time. Further, the optical sensor does not substantially degrade or deplete the sample as it does not affect the sample save to remove a very small amount of the analyte therefrom. Accordingly, the sensor may be placed in contact with a biological sample (such as saliva or blood) which is inside the body or a patient or more usually is removed from and returned to the body of a patient. All these factors mean that the sensor is extremely well-suited to perform continuous measurements on a sample, particularly a biological sample, for long periods of time.

Accordingly, in some embodiments, the method of measuring the CO₂ content of a sample is a method of continuously measuring the CO₂ content of the sample, wherein:
- the optical sensor is continuously exposed to the sample for an exposure period of at least ten minutes;
- excitation light is provided to the sensing region continuously or intermittently throughout the exposure period through the optical waveguide; and
- the intensity I₁ of emission of the fluorophore at a first wavelength λ₁, and optionally the intensity I₂ of emission of the fluorophore at a second wavelength λ₂, is/are detected continuously or intermittently throughout the exposure period through the optical waveguide.

The exposure period is preferably at least 1 hour. For instance, the exposure period may be at least 100 hours, typically up to 144 hours (7 days). The measurement method may therefore be used where it is important to monitor a biological sample (such as blood) for long periods, for instance during dialysis or open-heart surgery.

### Example

A positively-charged counter-ion moiety covalently bound to a polymeric support was produced as follows.

### Step (a)

Styrene (1.0 mass eq) and vinylbenzyl chloride (0.052 mass eq) were dissolved in toluene (55.7 vol). The solution was stirred and nitrogen bubbled through the solution for 30 minutes using a nitrogen dip tube. The nitrogen dip tube was then removed and the initiator AIBN (0.002 mass eq) was added; the solution was then heated to 60 °C for 18 hours. The resulting polymer, poly(styrene-co-vinylbenzyl chloride) was then precipitated out of solution by addition of methanol to give crude material which was twice dissolved in chloroform and precipitated with methanol. The resultant precipitate was then dried under high vacuum.

### Step (b)

The poly(styrene-co-vinylbenzyl chloride) (1.0 eq), a polymeric support, was dissolved in anhydrous DMF (20 vol) and stirred under nitrogen until fully dissolved. 45% aqueous trimethylamine (4.4 vol), a counter-ion moiety precursor, was added and the resulting mixture stirred at RT overnight. After stirring for 20 hr, an IPC was taken which was consistent with the formation of a new product. Solvents were removed under vacuum at 60°C. The resultant product was poly(styrene-co-(vinylbenzyl)trimethylammonium hydroxide), containing 5wt% vinylbenzyl)trimethylammonium hydroxide).

### Step (c)

The poly(styrene-co-(vinylbenzyl)trimethylammonium hydroxide) (500 mg) was dissolved in DCM (50 ml) and washed 3 times with NaOH (0.1 M, 50 ml). The solvent was then removed under vacuum

A reaction scheme involving steps (a) to (c) is shown below.

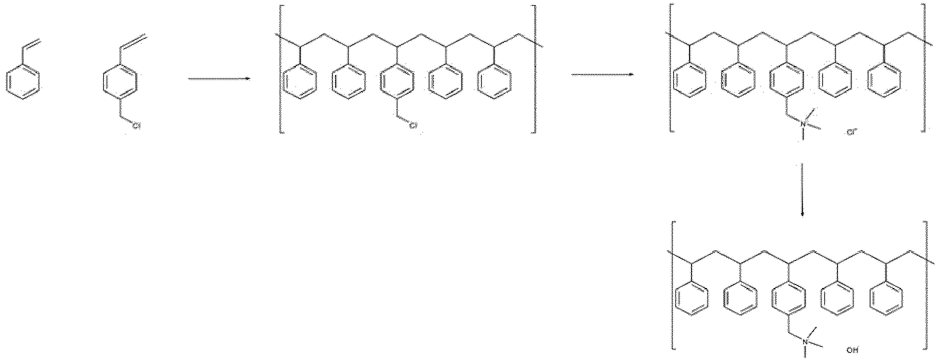

A polymer matrix further comprising a fluorophore moiety was then prepared by dissolving Poly(styrene-co-(vinylbenzyl)trimethylammonium hydroxide) (5wt% vinylbenzyl)trimethylammonium hydroxide) (30 mg) in dichloromethane (600 µl). The fluorophore moiety HTPS (2 µl, 10 mg.ml⁻¹ in water) was added and the mixture was shaken resulting in the HTPS being extracted in the organic phase.

A sensor was then prepared by dipping the tip of an optical waveguide (specifically an optical fibre) into this solution and allowing the solution to dry, forming a polymer matrix on the tip of the optical fibre. The sensor was tested by being placed in an environment containing CO₂.

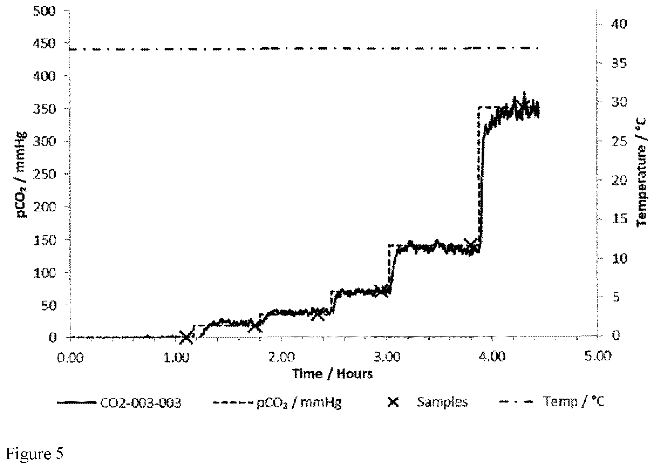

Binding curves and performance data from these fibres can be seen in Figures 4 and 5.

Figure 4 is a binding curve showing the binding of CO₂ to a polymer matrix in a sensor of the invention as a function of pCO₂. The proportion of CO₂ which binds to the matrix matches very closely to the predicted values, showing that the sensor efficaciously detects CO₂.

Figure 5 shows the actual pCO₂ values and pCO₂ values measured by a sensor according to the invention over a period of five hours. Also shown in this figure is the temperature, which was unchanged between 35 and 40 °C over the test period. It is evident that the actual pCO₂ value (partial pressure of CO₂ in the sample under test), shown by a dotted line, matches closely with the value detected using the sensor of the invention (shown with a solid line).

The accuracy of the sensor according to the invention is excellent. It was found that the sensor could detect pCO₂ values over the tested range (0.0 to 349.3 mmHg, 0-50%) with an accuracy of within 3.67%.

## Claims

1. A polymer matrix for optical sensing of CO₂, comprising a fluorophore, a positively charged counter-ion moiety and a polymeric support, wherein either the fluorophore or the counter-ion moiety is covalently bound to the polymeric support by a polymer linking moiety, and the molar ratio of counter-ion to fluorophore is 2:1 or greater.

2. The polymer matrix according to claim 1 wherein the fluorophore comprises a negatively-charged moiety at near-neutral pH, preferably wherein the fluorophore comprises an -O⁻ group or a -COO⁻ group at near-neutral pH.

3. The polymer matrix according to claim 1 or 2, wherein whichever of the fluorophore or the counter-ion moiety is not covalently bound to the polymeric support by a polymer linking moiety is suspended in the polymeric support.

4. The polymer matrix according to any preceding claim, wherein the fluorophore has a pH-dependent fluorescent emission spectrum.

5. The polymer matrix according to any preceding claim wherein the fluorophore comprises a species of formula (Fl-A): 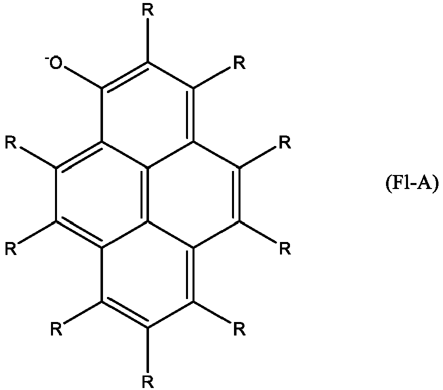
wherein each R group is independently selected from H, halogen, cyano, nitro, hydroxy, C₁₋₁₀ alkyl, C₃₋₁₀ cycloalkyl, C₄₋₁₀ cycloalkenyl, aryl, alkylaryl, heteroaryl, heterocycloalkyl, C₂₋₁₀ alkenyl, C₂₋₁₀ alkynyl, -SR^{a}, -OR^{a}, -SO₂R^{a}, -SO₃⁻, -SOR^{a}, - SO₂NR^{a}, -S(O)(NR^{a})R^{a}, -NR^{a}₂, -NR^{a}COR^{a}, -NR^{a}CO₂R^{a}, -COR^{a}, -CO₂R^{a}, -CONR^{a}₂, or a covalent bond attaching the fluorophore to the polymer linking moiety; or two neighbouring R groups are joined together to form a carbocyclic or heterocyclic ring;
each R^{a} group being independently selected from H, C₁₋₁₀ alkyl, C₃₋₁₀ cycloalkyl, C₄₋₁₀ cycloalkenyl, aryl, arylalkyl, heteroaryl, heterocycloalkyl, C₂₋₁₀ alkenyl, and C₂₋₁₀ alkynyl;
and any R group capable of substitution may be optionally substituted by one or more substituents independently selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -OR^{b}, - NR^{b}₂, -COR^{b}, -CO₂R^{b}, and -CONR^{b}₂,
wherein R^{b} is selected from H, C₁₋₆ alkyl, C₃₋₆ cycloalkyl, C₄₋₆ cycloalkenyl, and C₂₋₆ alkenyl;
or a species of formula (Fl-B) 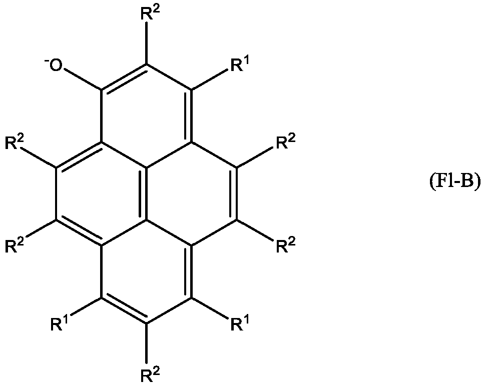
wherein each R¹ group is independently selected from halogen, cyano, nitro, hydroxy, -OR^{a}, -SO₂R^{a}, -SO₃⁻, -SOR^{a}, -SO₂NR^{a}, -S(O)(NR^{a})R^{a}, -NR^{a}₂, -NR^{a}COR^{a}, - NR^{a}CO₂R^{a}, -COR^{a}, -CO₂R^{a}, -CONR^{a}₂ and a covalent bond to the polymer linking moiety;
each R² group is independently selected from H, C₁₋₁₀ alkyl, C₃₋₁₀ cycloalkyl, C₄₋₁₀ cycloalkenyl, aryl, alkylaryl, heteroaryl, heterocycloalkyl, C₂₋₁₀ alkenyl, C₂₋₁₀ alkynyl, and a covalent bond to the polymer linking moiety;
any R¹ or R² group which is capable of substitution may be optionally substituted by one or more substituents independently selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -OR^{b}, -NR^{b}₂, -COR^{b}, -CO₂R^{b}, and -CONR^{b}₂; and
R^{a} and R^{b} are as defined above;
preferably wherein the fluorophore is pyranine, or a derivative thereof.

6. The polymer matrix according to any preceding claim wherein the counter-ion moiety is covalently bound to the polymeric support.

7. The polymer matrix according to any preceding claim wherein the counter-ion moiety comprises a quaternary ammonium cation, preferably a quaternary ammonium cation of formula (CI-A): 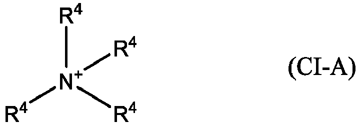
wherein each R⁴ group is independently selected from H; or C₁₋₃₀ alkyl, C₃₋₁₂ cycloalkyl, C₄₋₁₂ cycloalkenyl, aryl, alkylaryl, heteroaryl, heterocycloalkyl, C₂₋₃₀ alkenyl, C₂₋₃₀ alkynyl, and a covalent bond to the polymer linking moiety; or two R⁴ groups may be joined together to form a C₃₋₁₆ heterocycloalkyl or a C₄₋₁₆ heterocycloalkenyl group;
and any R⁴ group capable of substitution may be optionally substituted by one or more substituents independently selected from halogen, oxo, cyano, nitro, hydroxy, C₁₋₁₀ alkyl, C₃₋₁₀ cycloalkyl, C₄₋₁₀ cycloalkenyl, C₂₋₁₀ alkenyl, C₂₋₁₀ alkynyl, -OR^{b}, - NR^{b}₂, -COR^{b}, and -CO₂R^{b},
wherein R^{b} is as defined in claim 5;
more preferably wherein the counter-ion moiety is a hexadecyltrimethylammonium ion or a derivative thereof.

8. The polymer matrix according to claim 7 wherein one R⁴ group is a covalent bond to the polymer linking moiety and the remaining three R⁴ groups are each independently selected from H, C₁₋₄ alkyl and C₂₋₄ alkenyl.

9. The polymer matrix according to any preceding claim wherein the polymeric support is gas-permeable.

10. The polymer matrix according to any preceding claim wherein the polymeric support comprises
(a) a hydrophilic polymer, preferably selected from one or more of a hydrogel, a cellulose derivative, ethyl cellulose, a sol-gel, or a hydrophilic silicone-based polymer; particularly preferably selected from polyacrylamide or polyhydroxyethyl methacrylate; or
(b) a hydrophobic polymer, preferably selected from one or more of polystyrene, a hydrophobic silicone-based polymer, a hydrophobic cellulose derivative, or plasticised ethyl cellulose.

11. The polymer matrix according to any preceding claim wherein the polymer linking moiety comprises one or more of a covalent bond, -O-, -S-, -SO₂-, -SO-, an optionally substituted alkylene, an optionally substituted alkenylene, an optionally substituted alkynylene, and -NR⁵;
R⁵ is selected from H, C₁₋₁₀ alkyl, C₃₋₁₀ cycloalkyl, C₄₋₁₀ cycloalkenyl, aryl, arylalkyl, heteroaryl, heterocycloalkyl, C₂₋₁₀ alkenyl, C₂₋₁₀ alkynyl, -COR^{a}, -CO₂R^{a}, and - CONR^{a}₂; and
R^{a} is as defined in claim 5.

12. The polymer matrix according to any preceding claim wherein the polymer matrix also comprises water.

13. An optical sensor for optical sensing of CO₂, comprising:
- a sensing region comprising a polymer matrix as defined in any one of claims 1-12; and
- an optical waveguide arranged to direct light onto the sensing region.

14. The optical sensor according to claim 13 wherein the sensing region further comprises a hydrophobic membrane, and the sensor is configured to allow an analyte to enter the sensing region through the hydrophobic membrane.

15. The optical sensor according to claim 13 or claim 14, wherein the polymeric support comprises a hydrophilic polymer; the sensing region further comprises a gas-permeable hydrophobic membrane which is impermeable to liquids and hydrogen ions; and the sensor is configured to allow CO₂ to enter the sensing region through the hydrophobic membrane.

## Patentansprüche

1. Polymermatrix zur optischen Erfassung von CO₂, umfassend einen Fluorophor, eine positiv geladene Gegenionen-Komponente und einen Polymerträger, wobei entweder der Fluorophor oder die Gegenionen-Komponente durch eine Polymerverknüpfungskomponente kovalent an den Polymerträger gebunden ist und das molare Verhältnis von Gegenion zu Fluorophor 2:1 oder größer ist.

2. Polymermatrix nach Anspruch 1, wobei der Fluorophor eine negativ geladene Einheit bei nahezu neutralem pH-Wert umfasst, vorzugsweise wobei der Fluorophor eine -O⁻ Gruppe oder eine -COO⁻ Gruppe bei nahezu neutralem pH-Wert umfasst.

3. Polymermatrix nach Anspruch 1 oder 2, wobei der Fluorophor oder die Gegenionenkomponente, das/die durch eine Polymerverknüpfungskomponente nicht kovalent an den Polymerträger gebunden ist, in dem Polymerträger suspendiert ist.

4. Polymermatrix nach einem der vorhergehenden Ansprüche, wobei der Fluorophor ein pH-abhängiges Fluoreszenzemissionsspektrum aufweist.

5. Polymermatrix nach einem der vorhergehenden Ansprüche, wobei der Fluorophor eine Spezies der Formel (F1-A) umfasst: 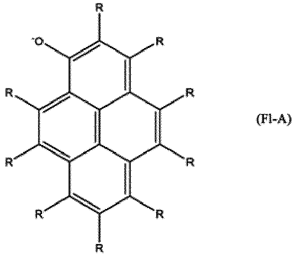
wobei jede R-Gruppe unabhängig ausgewählt ist aus H, Halogen, Cyano, Nitro, Hydroxy, C₁₋₁₀-Alkyl, C₃₋₁₀-Cycloalkyl, C₄₋₁₀-Cycloalkenyl, Aryl, Alkylaryl, Heteroaryl, Heterocycloalkyl, C₂₋₁₀-Alkenyl, C₂₋₁₀-Alkinyl, -SR^{a}, -OR^{a}, -SO₂R^{a}, -SO₃⁻, -SOR^{a}, - SO₂NR^{a}, -S(O)(NR^{a})R^{a}, -NR^{a}₂, -NR^{a}CoR^{a}, -NR^{a}CO₂R^{a}, -COR^{a}, -CO₂R^{a}, -CONR^{a}₂ oder einer kovalenten Bindung, die den Fluorophor an die Polymerverknüpfungseinheit bindet; oder wobei zwei benachbarte R-Gruppen miteinander verbunden sind, um einen carbocyclischen oder heterocyclischen Ring zu bilden;
wobei jede R^{a}-Gruppe unabhängig ausgewählt ist aus H, C₁₋₁₀-Alkyl, C₃₋₁₀-Cycloalkyl, C₄₋₁₀-Cycloalkenyl, Aryl, Arylalkyl, Heteroaryl, Heterocycloalkyl, C₂₋₁₀-Alkenyl und C₂₋₁₀-Alkinyl;
und jede R-Gruppe, die zur Substitution fähig ist, optional durch einen oder mehrere Substituenten substituiert sein kann, die unabhängig voneinander ausgewählt sind aus Halogen, Oxo, Cyano, Nitro, Hydroxy, C₁₋₁₀-Alkyl, C₃₋₁₀-Cycloalkyl, C₄₋₁₀-Cycloalkenyl, C₂₋₁₀-Alkenyl, C₂₋₁₀-Alkinyl, -OR^{b}, - NR^{b}₂, -COR^{b}, -CO₂R^{b} und -CONR^{b}₂,
wobei R_{b} ausgewählt ist aus H, C₁₋₆-Alkyl, C₃₋₆-Cycloalkyl, C₄₋₆-Cycloalkenyl und C₂₋₆-Alkenyl;
oder eine Spezies der Formel (F1-B) 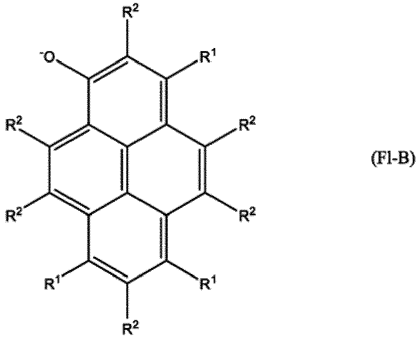
wobei jede R¹-Gruppe unabhängig ausgewählt ist aus Halogen, Cyano, Nitro, Hydroxy, -OR^{a}, -SO₂R^{a}, -SO₃⁻, -SOR^{a}, -SO₂NR^{a}, -S(O)(NR^{a})R^{a}, -NR^{a}₂, -NR^{a}COR^{a}, - NR^{a}CO₂R^{a}, -COR^{a}, -CO₂R^{a}, -CONR^{a}₂ und einer kovalenten Bindung an die Polymerverknüpfungskomponente;
jede R²-Gruppe unabhängig ausgewählt ist aus H, C₁₋₁₀-Alkyl, C₃₋₁₀-Cycloalkyl, C₄₋₁₀-Cycloalkenyl, Aryl, Alkylaryl, Heteroaryl, Heterocycloalkyl, C₂₋₁₀-Alkenyl, C₂₋₁₀-Alkinyl und einer kovalenten Bindung an die Polymerverknüpfungskomponente;
jede R¹- oder R²-Gruppe, die zur Substitution fähig ist, optional durch einen oder mehrere Substituenten substituiert sein kann, die unabhängig voneinander ausgewählt sind aus Halogen, Oxo, Cyano, Nitro, Hydroxy, C₁₋₁₀-Alkyl, C₃₋₁₀-Cycloalkyl, C₄₋₁₀-Cycloalkenyl, C₂₋₁₀-Alkenyl, C₂₋₁₀-Alkinyl, -OR^{b}, -NR^{b}₂, -COR^{b}, -CO₂R^{b} und -CONR^{b}₂; und
R^{a} und R^{b} wie oben definiert sind;
wobei der Fluorophor vorzugsweise Pyranin oder ein Derivat davon ist.

6. Polymermatrix nach einem der vorhergehenden Ansprüche, wobei die Gegenionen-Komponente kovalent an den Polymerträger gebunden ist.

7. Polymermatrix nach einem der vorhergehenden Ansprüche, wobei die Gegenionen-Komponente ein quaternäres Ammoniumkation, vorzugsweise ein quaternäres Ammoniumkation der Formel (CI-A), umfasst: 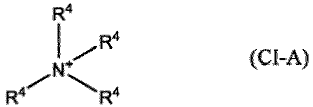
wobei jede R⁴-Gruppe unabhängig ausgewählt ist aus H; oder C₁₋₃₀-Alkyl, C₃₋₁₂-Cycloalkyl, C₄₋₁₂-Cycloalkenyl, Aryl, Alkylaryl, Heteroaryl, Heterocycloalkyl, C₂₋₃₀-Alkenyl, C₂₋₃₀-Alkinyl und einer kovalenten Bindung an die Polymerverknüpfungseinheit; oder wobei zwei R⁴-Gruppen miteinander verbunden sein können, um eine C₃₋₁₆-Heterocycloalkyl- oder eine C₄₋₁₆-Heterocycloalkenylgruppe zu bilden;
und jede R⁴-Gruppe, die zur Substitution fähig ist, optional durch einen oder mehrere Substituenten substituiert sein kann, die unabhängig voneinander ausgewählt sind aus Halogen, Oxo, Cyano, Nitro, Hydroxy, C₁₋₁₀-Alkyl, C₃₋₁₀-Cycloalkyl, C₄₋₁₀-Cycloalkenyl, C₂₋₁₀-Alkenyl, C₂₋₁₀-Alkinyl, -OR^{b}, -NR^{b}₂, -COR^{b} und -CO₂R^{b},
wobei R^{b} wie in Anspruch 5 definiert ist;
wobei die Gegenionen-Komponente vorzugsweise ein Hexadecyltrimethylammoniumion oder ein Derivat davon ist.

8. Polymermatrix nach Anspruch 7, wobei eine R⁴-Gruppe eine kovalente Bindung an die Polymerverknüpfungseinheit ist und die verbleibenden drei R⁴-Gruppen jeweils unabhängig voneinander aus H, C₁₋₄-Alkyl und C₂₋₄-Alkenyl ausgewählt sind.

9. Polymermatrix nach einem der vorhergehenden Ansprüche, wobei der Polymerträger gasdurchlässig ist.

10. Polymermatrix nach einem der vorhergehenden Ansprüche, wobei der Polymerträger Folgendes umfasst
(a) ein hydrophiles Polymer, vorzugsweise ausgewählt aus einem oder mehreren von einem Hydrogel, einem Cellulosederivat, Ethylcellulose, einem Sol-Gel oder einem hydrophilen Polymer auf Silikonbasis; besonders bevorzugt ausgewählt aus Polyacrylamid oder Polyhydroxyethylmethacrylat; oder
(b) ein hydrophobes Polymer, vorzugsweise ausgewählt aus einem oder mehreren von Polystyrol, einem hydrophoben Polymer auf Silikonbasis, einem hydrophoben Cellulosederivat oder plastifizierter Ethylcellulose.

11. Polymermatrix nach einem der vorhergehenden Ansprüche, wobei die Polymerverknüpfungskomponente eines oder mehrere von einer kovalenten Bindung, -O-, -S-, -SO₂-, -SO-, einem optional substituierten Alkylen, einem optional substituierten Alkenylen, einem optional substituierten Alkinylen und -NR⁵ umfasst;
R⁵ ausgewählt ist aus H, C₁₋₁₀-Alkyl, C₃₋₁₀-Cycloalkyl, C₄₋₁₀-Cycloalkenyl, Aryl, Arylalkyl, Heteroaryl, Heterocycloalkyl, C₂₋₁₀-Alkenyl, C₂₋₁₀-Alkinyl, -COR^{a}, -CO₂R^{a} und - CONR^{a}₂; und
R^{a} wie in Anspruch 5 definiert ist.

12. Polymermatrix nach einem der vorhergehenden Ansprüche, wobei die Polymermatrix auch Wasser umfasst.

13. Optischer Sensor zur optischen Erfassung von CO₂, umfassend:
- einen Erfassungsbereich, der eine Polymermatrix wie in einem der Ansprüche 1 bis 12 definiert umfasst; und
- einen optischen Wellenleiter, der so angeordnet ist, dass er Licht auf den Erfassungsbereich richtet.

14. Optischer Sensor nach Anspruch 13, wobei der Erfassungsbereich ferner eine hydrophobe Membran umfasst und der Sensor konfiguriert ist, um einen Analyten durch die hydrophobe Membran in den Erfassungsbereich eintreten zu lassen.

15. Optischer Sensor nach Anspruch 13 oder 14, wobei der Polymerträger ein hydrophiles Polymer umfasst; der Erfassungsbereich ferner eine gasdurchlässige hydrophobe Membran umfasst, die für Flüssigkeiten und Wasserstoffionen undurchlässig ist; und der Sensor konfiguriert ist, um den Eintritt von CO₂ in den Erfassungsbereich durch die hydrophobe Membran zu ermöglichen.

## Revendications

1. Matrice polymère pour détection optique de CO₂, comprenant un fluorophore, une fraction contre-ion positivement chargée et un support polymérique, dans laquelle soit le fluorophore soit la fraction contre-ion est lié de façon covalente au support polymérique par une fraction de couplage polymère, et le rapport molaire de contre-ion par rapport au fluorophore est 2 : 1 ou plus.

2. Matrice polymère selon la revendication 1, dans laquelle le fluorophore comprend une fraction négativement chargée à pH quasi-neutre, de préférence dans laquelle le fluorophore comprend un groupe -O⁻ ou un groupe -COO⁻ à pH quasi-neutre.

3. Matrice polymère selon la revendication 1 ou 2, dans laquelle l'un ou l'autre du fluorophore ou de la fraction contre-ion qui n'est pas lié de façon covalente au support polymérique par une fraction de couplage polymère est suspendu dans le support polymérique.

4. Matrice polymère selon une quelconque revendication précédente, dans laquelle le fluorophore a un spectre d'émission fluorescent dépendant du pH.

5. Matrice polymère selon une quelconque revendication précédente, dans laquelle le fluorophore comprend une espèce de la formule (F1-A) : 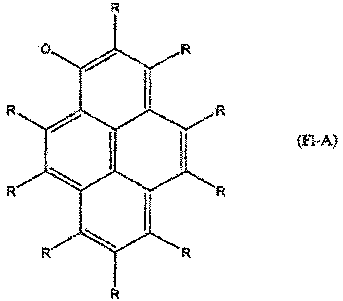
dans laquelle chaque groupe R est indépendamment sélectionné parmi H, halogène, cyano, nitro, hydroxy, alkyle en C₁₋₁₀, cycloalkyle en C₃₋₁₀, cycloalcényle en C₄₋₁₀, aryle, alkylaryle, hétéroaryle, hétérocycloalkyle, alcényle en C₂₋₁₀, alkynyle en C₂₋₁₀, -SR^{a}, -OR^{a}, -SO₂R^{a}, -SO₃⁻, -SOR^{a}, -SO₂NR^{a}, -S(O)(NR^{a})R^{a}, -NR^{a}₂, -NR^{a}COR^{a}, -NR^{a}CO₂R^{a}, -COR^{a}, -CO₂R^{a}, -CONR^{a}₂, ou une liaison covalente attachant le fluorophore à la fraction de couplage polymère ; ou deux groupes R voisins sont joints ensemble pour former un anneau carbocyclique ou hétérocyclique ;
chaque groupe R^{a} étant indépendamment sélectionné parmi H, alkyle en C₁₋₁₀, cycloalkyle en C₃₋₁₀, cycloalcényle en C₄₋₁₀, aryle, arylalkyle, hétéroaryle, hétérocycloalkyle, alcényle en C₂₋₁₀, et alkynyle en C₂₋₁₀ ;
et tout groupe R capable de substitution peut être facultativement substitué avec un ou plusieurs substituants indépendamment sélectionnés parmi halogène, oxo, cyano, nitro, hydroxy, alkyle en C₁₋₁₀, cycloalkyle en C₃₋₁₀, cycloalcényle en C₄₋₁₀, alcényle en C₂₋₁₀, alkynyle en C₂₋₁₀, -OR^{b}, -NR^{b}₂, -COR^{b}, -CO₂R^{b}, et -CONR^{b}₂,
dans laquelle R^{b} est sélectionné parmi H, alkyle en C₁₋₆, cycloalkyle en C₃₋₆, cycloalcényle en C₄₋₆, et alcényle en C₂₋₆ ;
ou une espèce de la formule (F1-B) 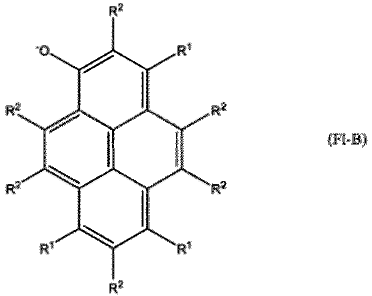
dans laquelle chaque groupe R¹ est indépendamment sélectionné parmi halogène, cyano, nitro, hydroxy, -OR^{a}, -SO₂R^{a}, -SO₃⁻, -SOR^{a}, -SO₂NR^{a}, -S(O)(NR^{a})R^{a}, -NR^{a}₂, -NR^{a}COR^{a}, -NR^{a}CO₂R^{a}, -COR^{a}, -CO₂R^{a}, -CONR^{a}₂ et une liaison covalente à la fraction de couplage polymère ;
chaque groupe R² est indépendamment sélectionné parmi H, alkyle en C₁₋₁₀, cycloalkyle en C₃₋₁₀, cycloalcényle en C₄₋₁₀, aryle, alkylaryle, hétéroaryle, hétérocycloalkyle, alcényle en C₂₋₁₀, alkynyle en C₂₋₁₀, et une liaison covalente à la fraction de couplage polymère ;
tout groupe R¹ ou R² qui est capable de substitution peut être facultativement substitué avec un ou plusieurs substituants indépendamment sélectionnés parmi halogène, oxo, cyano, nitro, hydroxy, alkyle en C₁₋₁₀, cycloalkyle en C₃₋₁₀, cycloalcényle en C₄₋₁₀, alcényle en C₂₋₁₀, alkynyle en C₂₋₁₀, -OR^{b}, -NR^{b}₂, -COR^{b}, -CO₂R^{b}, et -CONR^{b}₂ ; et
R^{a} et R^{b} sont tels que définis ci-dessus ;
de préférence dans laquelle le fluorophore est pyranine, ou un dérivé de celle-ci.

6. Matrice polymère selon une quelconque revendication précédente, dans laquelle la fraction contre-ion est liée de façon covalente au support polymérique.

7. Matrice polymère selon une quelconque revendication précédente, dans laquelle la fraction contre-ion comprend un cation ammonium quaternaire, de préférence un cation ammonium quaternaire de la formule (CI-A) : 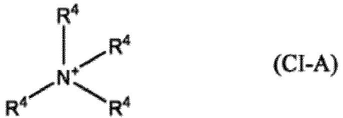
dans laquelle chaque groupe R⁴ est indépendamment sélectionné parmi H ; ou alkyle en C₁₋₃₀, cycloalkyle en C₃₋₁₂, cycloalcényle en C₄₋₁₂, aryle, alkylaryle, hétéroaryle, hétérocycloalkyle, alcényle en C₂₋₃₀, alkynyle en C₂₋₃₀, et une liaison covalente à la fraction de couplage polymère ; ou deux groupes R⁴ peuvent être joints ensemble pour former un hétérocycloalkyle en C₃₋₁₆ ou un groupe hétérocycloalcényle en C₄₋₁₆ ;
et tout groupe R⁴ capable de substitution peut être facultativement substitué avec un ou plusieurs substituants indépendamment sélectionnés parmi halogène, oxo, cyano, nitro, hydroxy, alkyle en C₁₋₁₀, cycloalkyle en C₃₋₁₀, cycloalcényle en C₄₋₁₀, alcényle en C₂₋₁₀, alkynyle en C₂₋₁₀, -OR^{b}, -NR^{b}₂, -COR^{b}, et -CO₂R^{b},
dans laquelle R^{b} est tel que défini dans la revendication 5 ;
de façon davantage préférée dans laquelle la fraction contre-ion est un ion hexadécyltriméthylammonium ou un dérivé de celui-ci.

8. Matrice polymère selon la revendication 7 dans laquelle un groupe R⁴ est une liaison covalente à la fraction de couplage polymère et les trois autres groupes R⁴ sont chacun indépendamment sélectionnés parmi H, alkyle en C₁₋₄ et alcényle en C₂₋₄.

9. Matrice polymère selon une quelconque revendication précédente, dans laquelle le support polymérique est perméable aux gaz.

10. Matrice polymère selon une quelconque revendication précédente, dans laquelle le support polymérique comprend
(a) un polymère hydrophile, de préférence sélectionné parmi un ou plusieurs de : un hydrogel, un dérivé de cellulose, cellulose d'éthyle, un sol-gel, ou un polymère à base de silicone hydrophile ; de préférence particulière sélectionné parmi polyacrylamide ou méthacrylate de polyhydroxyéthyle ; ou
(b) un polymère hydrophobe, de préférence sélectionné parmi un ou plusieurs de : polystyrène, un polymère à base de silicone hydrophobe, un hydrophobe dérivé de cellulose, ou une cellulose d'éthyle plastifiée.

11. Matrice polymère selon une quelconque revendication précédente, dans laquelle la fraction de couplage polymère comprend un ou plusieurs de : une liaison covalente, -O-, -S-, -SO₂-, -SO-, un alkylène facultativement substitué, un alcénylène facultativement substitué, un alkynylène facultativement substitué, et -NR⁵ ;
R⁵ est sélectionné parmi H, alkyle en C₁₋₁₀, cycloalkyle en C₃₋₁₀, cycloalcényle en C₄₋₁₀, aryle, arylalkyle, hétéroaryle, hétérocycloalkyle, alcényle en C₂₋₁₀, alkynyle en C₂₋₁₀, -COR^{a}, -CO₂R^{a}, et -CONR^{a}₂ ; et
R^{a} est tel que défini dans la revendication 5.

12. Matrice polymère selon une quelconque revendication précédente, dans laquelle la matrice polymère comprend également de l'eau.

13. Capteur optique pour détection optique de CO₂, comprenant :
- une région de détection comprenant une matrice polymère telle que définie dans l'une quelconque des revendications 1 à 12 ; et
- un guide d'ondes optique agencé pour diriger de la lumière sur la région de détection.

14. Capteur optique selon la revendication 13, dans lequel la région de détection comprend en outre une membrane hydrophobe, et le capteur est configuré pour permettre à un analyte d'entrer dans la région de détection à travers la membrane hydrophobe.

15. Capteur optique selon la revendication 13 ou la revendication 14, dans lequel le support polymérique comprend un polymère hydrophile ; la région de détection comprend en outre une membrane hydrophobe perméable aux gaz qui est imperméable aux liquides et ions d'hydrogène ; et le capteur est configuré pour permettre à CO₂ d'entrer dans la région de détection à travers la membrane hydrophobe.
